# EUROPEAN PATENT APPLICATION

(11) **EP 2 829 601 A1**
(43) Date of publication of application: **28.01.2015**
(21) Application number: 14178739.0
(22) Date of filing: 28.07.2014
(51) Int. Cl.: C12N 9/02, C12N 9/10, C12N 9/88, C12P 7/46

(54) **Bacterial cell having enhanced succinic acid production and a method for producing the succinic acid using the same**

(30) Priority: 26.07.2013 KR 20130088972
(71) Applicant: Samsung Electronics Co., Ltd, Gyeonggi-do 443-742 (KR)
(72) Inventor: Park, Joonsong, 443-803 Gyeonggi-do (KR); Park, Jinhwan, 443-803 Gyeonggi-do (KR); Chung, Soonchun, 443-803 Gyeonggi-do (KR); Yun, Jiae, 443-803 Gyeonggi-do (KR); Cho, Kwangmyung, 443-803 Gyeonggi-do (KR)
(74) Representative: Grünecker, Kinkeldey, Stockmair & Schwanhäusser

(57) **Abstract**

A genetically engineered bacterial cell wherein activity of a pathway in the cell of converting α-ketoglutarate into succinate semialdehyde; or activity of succinyl semialdehyde dehydrogenase in the cell is increased compared to the activity in a non-genetically engineered cell of the same type, and a method of producing succinic acid by using the same.

<110>    SAMSUNG ELECTORNICS CO., LTD.

<120>    Bacteria cell having enhanced succinic acid production and a
         method for producing the succinic acid using the same

<130>    EP95116FZTRpau

<140>    not yet assigned
<141>    herewith

<150>    10-2013-0088972
<151>    2013-07-26

<160>    69

<170>    KopatentIn 2.0

<210>    1
<211>    543
<212>    PRT
<213>    Corynebacterium glutamicum

<400>    1
Met Ser Ser Thr Pro Ala Gln Asp Leu Ala Arg Ala Val Ile Asp Ser
  1               5                  10                  15

Leu Ala Pro His Val Thr Asp Val Val Leu Cys Pro Gly Ser Arg Asn
             20                  25                  30

Ser Pro Leu Ser Leu Glu Leu Leu Ala Arg Gln Asp Leu Arg Val His
         35                  40                  45

Val Arg Ile Asp Glu Arg Ser Ala Ser Phe Leu Ala Leu Ser Leu Ala
     50                  55                  60

Arg Thr Gln Ala Arg Pro Val Ala Val Val Met Thr Ser Gly Thr Ala
 65                  70                  75                  80

Val Ala Asn Cys Leu Pro Ala Val Ala Glu Ala Ala His Ala His Ile
                 85                  90                  95

Pro Leu Ile Val Leu Ser Ala Asp Arg Pro Ala His Leu Val Gly Thr
            100                 105                 110

Gly Ala Ser Gln Thr Ile Asn Gln Thr Gly Ile Phe Gly Asp Leu Ala
        115                 120                 125

Pro Thr Val Gly Ile Thr Glu Leu Asp Gln Val Ala Gln Ile Ala Glu
    130                 135                 140

Ser Leu Ala Gln Gly Ala Ser Gln Ile Pro Arg His Phe Asn Leu Ala
145                 150                 155                 160

Leu Asp Val Pro Leu Val Ala Pro Glu Leu Pro Glu Leu His Gly Glu
                165                 170                 175

Ala Val Gly Ala Ser Trp Thr His Arg Trp Ile Asn His Gly Glu Val
            180                 185                 190

Thr Val Asp Leu Gly Glu His Thr Leu Val Ile Ala Gly Asp Glu Ala
        195                 200                 205

Trp Glu Val Glu Gly Leu Glu Asp Val Pro Thr Ile Ala Glu Pro Thr

210                 215                 220

Ala Pro Lys Pro Tyr Asn Pro Val His Pro Leu Ala Ala Glu Ile Leu
225                 230                 235                 240

Leu Lys Glu Gln Val Ser Ala Glu Gly Tyr Val Val Asn Thr Arg Pro
                245                 250                 255

Asp His Val Ile Val Val Gly His Pro Thr Leu His Arg Gly Val Leu
            260                 265                 270

Lys Leu Met Ser Asp Pro Gly Ile Lys Leu Thr Val Leu Ser Arg Thr
        275                 280                 285

Asp Ile Ile Thr Asp Pro Gly Arg His Ala Asp Gln Val Gly Ser Thr
    290                 295                 300

Val Lys Val Thr Gly Thr Gln Glu Lys Gln Trp Leu Lys Ile Cys Ser
305                 310                 315                 320

Ala Ala Ser Glu Leu Ala Ala Asp Gly Val Arg Asp Val Leu Asp Asn
                325                 330                 335

Gln Glu Phe Gly Phe Thr Gly Leu His Val Ala Ala Ala Val Ala Asp
            340                 345                 350

Thr Leu Gly Thr Gly Asp Thr Leu Phe Ala Ala Ala Ser Asn Ser Ile
        355                 360                 365

Arg Asp Leu Ser Leu Val Gly Met Pro Phe Asp Gly Val Asp Thr Phe
    370                 375                 380

Ser Pro Arg Gly Val Ala Gly Ile Asp Gly Ser Val Ala Gln Ala Ile
385                 390                 395                 400

Gly Thr Ser Leu Ala Val Gln Ser Arg His Pro Asp Glu Ile Arg Ala
                405                 410                 415

Pro Arg Thr Val Ala Leu Leu Gly Asp Leu Ser Phe Leu His Asp Ile
            420                 425                 430

Gly Gly Leu Leu Ile Gly Pro Asp Glu Pro Arg Pro Glu Asn Leu Thr
        435                 440                 445

Ile Val Val Ser Asn Asp Asn Gly Gly Gly Ile Phe Glu Leu Leu Glu
    450                 455                 460

Thr Gly Ala Asp Gly Leu Arg Pro Asn Phe Glu Arg Ala Phe Gly Thr
465                 470                 475                 480

Pro His Asp Ala Ser Ile Ala Asp Leu Cys Ala Gly Tyr Gly Ile Glu
                485                 490                 495

His Gln Val Val Asp Asn Leu Gln Asp Leu Ile Ile Ala Leu Val Asp
            500                 505                 510

Thr Thr Glu Val Ser Gly Phe Thr Ile Ile Glu Ala Ser Thr Val Arg
        515                 520                 525

Asp Thr Arg Arg Ala Gln Gln Gln Ala Leu Met Asp Thr Val His
    530                 535                 540

<210>    2
<211>    933
<212>    PRT
<213>    Escherichia coli

<400>    2
Met Gln Asn Ser Ala Leu Lys Ala Trp Leu Asp Ser Ser Tyr Leu Ser
1               5                   10                  15

Gly Ala Asn Gln Ser Trp Ile Glu Gln Leu Tyr Glu Asp Phe Leu Thr
            20                  25                  30

Asp Pro Asp Ser Val Asp Ala Asn Trp Arg Ser Thr Phe Gln Gln Leu
        35                  40                  45

Pro Gly Thr Gly Val Lys Pro Asp Gln Phe His Ser Gln Thr Arg Glu
    50                  55                  60

Tyr Phe Arg Arg Leu Ala Lys Asp Ala Ser Arg Tyr Ser Ser Thr Ile
65                  70                  75                  80

Ser Asp Pro Asp Thr Asn Val Lys Gln Val Lys Val Leu Gln Leu Ile
                85                  90                  95

Asn Ala Tyr Arg Phe Arg Gly His Gln His Ala Asn Leu Asp Pro Leu
            100                 105                 110

Gly Leu Trp Gln Gln Asp Lys Val Ala Asp Leu Asp Pro Ser Phe His
        115                 120                 125

Asp Leu Thr Glu Ala Asp Phe Gln Glu Thr Phe Asn Val Gly Ser Phe
    130                 135                 140

Ala Ser Gly Lys Glu Thr Met Lys Leu Gly Glu Leu Leu Glu Ala Leu
145                 150                 155                 160

Lys Gln Thr Tyr Cys Gly Pro Ile Gly Ala Glu Tyr Met His Ile Thr
                165                 170                 175

Ser Thr Glu Glu Lys Arg Trp Ile Gln Gln Arg Ile Glu Ser Gly Arg
            180                 185                 190

Ala Thr Phe Asn Ser Glu Glu Lys Lys Arg Phe Leu Ser Glu Leu Thr
        195                 200                 205

Ala Ala Glu Gly Leu Glu Arg Tyr Leu Gly Ala Lys Phe Pro Gly Ala
    210                 215                 220

Lys Arg Phe Ser Leu Glu Gly Gly Asp Ala Leu Ile Pro Met Leu Lys
225                 230                 235                 240

Glu Met Ile Arg His Ala Gly Asn Ser Gly Thr Arg Glu Val Val Leu
                245                 250                 255

Gly Met Ala His Arg Gly Arg Leu Asn Val Leu Val Asn Val Leu Gly
            260                 265                 270

Lys Lys Pro Gln Asp Leu Phe Asp Glu Phe Ala Gly Lys His Lys Glu
        275                 280                 285

His Leu Gly Thr Gly Asp Val Lys Tyr His Met Gly Phe Ser Ser Asp

290                 295                 300

Phe Gln Thr Asp Gly Gly Leu Val His Leu Ala Leu Ala Phe Asn Pro
305                 310                 315                 320

Ser His Leu Glu Ile Val Ser Pro Val Val Ile Gly Ser Val Arg Ala
                325                 330                 335

Arg Leu Asp Arg Leu Asp Glu Pro Ser Ser Asn Lys Val Leu Pro Ile
            340                 345                 350

Thr Ile His Gly Asp Ala Ala Val Thr Gly Gln Gly Val Val Gln Glu
        355                 360                 365

Thr Leu Asn Met Ser Lys Ala Arg Gly Tyr Glu Val Gly Gly Thr Val
    370                 375                 380

Arg Ile Val Ile Asn Asn Gln Val Gly Phe Thr Thr Ser Asn Pro Leu
385                 390                 395                 400

Asp Ala Arg Ser Thr Pro Tyr Cys Thr Asp Ile Gly Lys Met Val Gln
                405                 410                 415

Ala Pro Ile Phe His Val Asn Ala Asp Asp Pro Glu Ala Val Ala Phe
            420                 425                 430

Val Thr Arg Leu Ala Leu Asp Phe Arg Asn Thr Phe Lys Arg Asp Val
        435                 440                 445

Phe Ile Asp Leu Val Cys Tyr Arg Arg His Gly His Asn Glu Ala Asp
    450                 455                 460

Glu Pro Ser Ala Thr Gln Pro Leu Met Tyr Gln Lys Ile Lys Lys His
465                 470                 475                 480

Pro Thr Pro Arg Lys Ile Tyr Ala Asp Lys Leu Glu Gln Glu Lys Val
                485                 490                 495

Ala Thr Leu Glu Asp Ala Thr Glu Met Val Asn Leu Tyr Arg Asp Ala
            500                 505                 510

Leu Asp Ala Gly Asp Cys Val Val Ala Glu Trp Arg Pro Met Asn Met
        515                 520                 525

His Ser Phe Thr Trp Ser Pro Tyr Leu Asn His Glu Trp Asp Glu Glu
    530                 535                 540

Tyr Pro Asn Lys Val Glu Met Lys Arg Leu Gln Glu Leu Ala Lys Arg
545                 550                 555                 560

Ile Ser Thr Val Pro Glu Ala Val Glu Met Gln Ser Arg Val Ala Lys
                565                 570                 575

Ile Tyr Gly Asp Arg Gln Ala Met Ala Ala Gly Glu Lys Leu Phe Asp
            580                 585                 590

Trp Gly Gly Ala Glu Asn Leu Ala Tyr Ala Thr Leu Val Asp Glu Gly
        595                 600                 605

Ile Pro Val Arg Leu Ser Gly Glu Asp Ser Gly Arg Gly Thr Phe Phe
    610                 615                 620

His Arg His Ala Val Ile His Asn Gln Ser Asn Gly Ser Thr Tyr Thr

625                 630                 635                 640

Pro Leu Gln His Ile His Asn Gly Gln Gly Ala Phe Arg Val Trp Asp
                645                 650                 655

Ser Val Leu Ser Glu Glu Ala Val Leu Ala Phe Glu Tyr Gly Tyr Ala
            660                 665                 670

Thr Ala Glu Pro Arg Thr Leu Thr Ile Trp Glu Ala Gln Phe Gly Asp
        675                 680                 685

Phe Ala Asn Gly Ala Gln Val Val Ile Asp Gln Phe Ile Ser Ser Gly
    690                 695                 700

Glu Gln Lys Trp Gly Arg Met Cys Gly Leu Val Met Leu Leu Pro His
705                 710                 715                 720

Gly Tyr Glu Gly Gln Gly Pro Glu His Ser Ser Ala Arg Leu Glu Arg
                725                 730                 735

Tyr Leu Gln Leu Cys Ala Glu Gln Asn Met Gln Val Cys Val Pro Ser
            740                 745                 750

Thr Pro Ala Gln Val Tyr His Met Leu Arg Arg Gln Ala Leu Arg Gly
        755                 760                 765

Met Arg Arg Pro Leu Val Val Met Ser Pro Lys Ser Leu Leu Arg His
    770                 775                 780

Pro Leu Ala Val Ser Ser Leu Glu Glu Leu Ala Asn Gly Thr Phe Leu
785                 790                 795                 800

Pro Ala Ile Gly Glu Ile Asp Glu Leu Asp Pro Lys Gly Val Lys Arg
                805                 810                 815

Val Val Met Cys Ser Gly Lys Val Tyr Tyr Asp Leu Leu Glu Gln Arg
            820                 825                 830

Arg Lys Asn Asn Gln His Asp Val Ala Ile Val Arg Ile Glu Gln Leu
        835                 840                 845

Tyr Pro Phe Pro His Lys Ala Met Gln Glu Val Leu Gln Gln Phe Ala
    850                 855                 860

His Val Lys Asp Phe Val Trp Cys Gln Glu Glu Pro Leu Asn Gln Gly
865                 870                 875                 880

Ala Trp Tyr Cys Ser Gln His His Phe Arg Glu Val Ile Pro Phe Gly
                885                 890                 895

Ala Ser Leu Arg Tyr Ala Gly Arg Pro Ala Ser Ala Ser Pro Ala Val
            900                 905                 910

Gly Tyr Met Ser Val His Gln Lys Gln Gln Gln Asp Leu Val Asn Asp
        915                 920                 925

Ala Leu Asn Val Glu
    930

<210>   3
<211>   1632
<212>   DNA

<213>    Corynebacterium glutamicum

<400>    3
atgtccagca cgccagctca agatcttgcc cgcgccgtta ttgattccct cgcaccacac    60

gtcactgacg tggtgttatg cccaggatcc aggaactcac cgttgtcgct tgagttgctg    120

gcgcggcagg atctgcgtgt ccatgtgcgt atcgacgagc gcagcgcctc atttttggcg    180

ctgtccctag cgcgtaccca ggcccggccg gtggctgtgg tgatgacctc cggcacggct    240

gtagctaact gcctgcctgc tgttgctgaa gctgcgcatg cccatatccc gttgattgtg    300

ctctctgctg accgtcctgc acatttggtg ggaacggggg cgagccaaac gattaaccag    360

accggtattt ttggtgatct tgcaccgacg gtcggtatca ctgagctgga tcaggtagcg    420

cagattgctg aaagccttgc tcagggggct tcccagattc cgcgtcattt caatcttgca    480

cttgatgttc ctttggttgc tcctgaactg ccagagcttc atggtgaggc agttggagca    540

tcatggacgc atcgctggat caaccacggt gaggtgaccg tggacctggg ggagcacacc    600

ctcgtgattg ccggtgatga agcatgggaa gtggaagggc tggaagatgt gcccaccatc    660

gctgaaccta ctgcaccaaa gccttataat ccggtgcacc cactggctgc tgaaatcttg    720

ctgaaggagc aggtctccgc ggaaggctat gtggtaaaca ccaggcctga tcatgtgatc    780

gtggtgggac accccacgct gcaccgcgga gtgttgaagt tgatgtcaga tcctggcatt    840

aaattaactg tgctttcacg caccgatatc atcactgatc ccggccgcca tgccgatcag    900

gtgggcagca cagtgaaagt caccggcacc caggaaaagc agtggctaaa gatctgttcg    960

gcagcatcag aacttgcggc cgatggtgtg cgtgacgtcc tggacaacca agaattcggt    1020

ttcaccggcc tccatgttgc cgcagccgtg gcggatacct taggcaccgg cgatactctc    1080

tttgctgcag catccaactc aatccgtgac ctctccctgg tgggtatgcc ttttgatggc    1140

gtggatacct tctccccacg aggtgtcgca ggcattgatg gttctgttgc tcaagcaatc    1200

ggcacttcac ttgctgtgca gtcccgccac cccgatgaaa tccgcgcgcc acgcactgtg    1260

gcccttctgg gcgatctgtc gttccttcac gatattggcg gactgctcat cggccctgat    1320

gaaccacgcc cagaaaacct caccatcgtg gtctccaacg acaacggtgg cggaatcttc    1380

gaactcctag aaaccggcgc agatggtctc cgccccaact tcgagcgtgc tttcggtacc    1440

ccacacgacg cgtccatcgc ggatctctgc gcaggctacg gcattgaaca ccaagtggta    1500

gacaacctcc aagacctcat catcgcgcta gttgatacca ccgaagtatc cggattcacc    1560

attattgaag cttcgaccgt ccgagatacc cgccgtgcac aacagcaagc tctcatggac    1620

acggtgcact aa    1632

<210>    4
<211>    2802
<212>    DNA

<213>    Escherichia coli

<400>    4
atgcagaaca gcgctttgaa agcctggttg gactcttctt acctctctgg cgcaaaccag      60

agctggatag aacagctcta tgaagacttc ttaaccgatc ctgactcggt tgacgctaac     120

tggcgttcga cgttccagca gttacctggt acgggagtca aaccggatca attccactct     180

caaacgcgtg aatatttccg ccgcctggcg aaagacgctt cacgttactc ttcaacgatc     240

tccgaccctg acaccaatgt gaagcaggtt aaagtcctgc agctcattaa cgcataccgc     300

ttccgtggtc accagcatgc gaatctcgat ccgctgggac tgtggcagca agataaagtg     360

gccgatctgg atccgtcttt ccacgatctg accgaagcag acttccagga gaccttcaac     420

gtcggttcat ttgccagcgg caaagaaacc atgaaactcg gcgaactgct ggaagcactc     480

aagcaaacct actgcggccc gattggtgcc gagtatatgc acattaccag cactgaagaa     540

aaacgctgga tccaacagcg tattgagtct ggtcgcgcga ctttcaatag cgaagagaaa     600

aaacgcttct taagcgaact gaccgccgct gaaggccttg aacgttacct cggcgcaaaa     660

ttccctggcg caaaacgctt ctcgctggaa ggcggtgacg cgttaatccc gatgcttaaa     720

gagatgatcc gccacgctgg caacagcggc acccgcgaag tggttctcgg aatggcgcac     780

cgtggtcgtc tgaacgtgct ggtgaacgtg ctgggtaaaa aaccgcaaga cttgttcgac     840

gagtttgccg gtaaacataa agaacacctc ggcacgggcg acgtgaaata ccacatgggc     900

ttctcgtctg acttccagac cgatggcggc ctggttcacc tggcgctggc gtttaacccg     960

tctcaccttg agattgtaag cccggtcgtt atcggttctg ttcgtgcccg tctggacaga    1020

cttgatgagc cgagcagcaa caaagtgctg ccaatcacca ttcatggtga cgccgcagtg    1080

accgggcagg gcgtggttca ggaaaccctg aacatgtcga aagcgcgtgg ttatgaagtt    1140

ggcggtacgg tacgtatcgt tatcaacaac caggttggct tcaccacctc taacccgctg    1200

gatgcccgtt cgacgccgta ctgtactgat atcggtaaga tggttcaggc accgattttc     1260

cacgttaacg cggatgatcc ggaagccgtt gcctttgtta cccgtctggc gctcgatttc    1320

cgtaacacct ttaaacgtga tgtcttcatc gacctggtat gctaccgccg tcacggccac    1380

aacgaagccg acgagccgag cgcaacccag ccgctgatgt atcagaaaat caaaaaacat    1440

ccgacgccgc gcaaaatcta cgctgacaag ctggagcagg aaaaagtcgc gacgctggaa    1500

gatgccaccg agatggttaa cctgtaccgc gatgcgctgg atgctggcga ttgcgttgta    1560

gcagagtggc gtccgatgaa catgcactct ttcacctggt cgccgtacct caaccatgaa    1620

tgggacgaag agtacccgaa caaagttgag atgaagcgcc tgcaggaact ggctaaacgc    1680

atcagcacgg tgccggaagc ggttgaaatg cagtctcgcg ttgccaagat ttatggcgat    1740

cgccaggcga tggcagccgg tgagaaactg ttcgactggg gcggcgcgga aaacctcgct    1800

tacgccacgt tggttgacga aggcattccg gttcgcctgt cgggtgaaga ctccggtcgc 1860

ggtaccttct tccaccgcca cgcggtgatc cacaaccagt ctaacggttc cacttacacg 1920

ccgctgcaac atatccataa cggccagggc gcgttccgcg tctgggactc tgtactgtct 1980

gaagaagccg tactggcgtt tgaatacggt tatgccaccg cagaaccacg caccctgacc 2040

atctgggaag cacagttcgg tgacttcgcc aacggtgcac aggtggttat cgaccagttc 2100

atctcctctg gcgaacagaa atggggccgg atgtgtggcc tggtgatgtt gctgccgcac 2160

ggttacgaag ggcaggggcc ggagcactcc tccgcgcgtc tggaacgtta tctgcaactt 2220

tgcgctgagc aaaacatgca ggtttgcgta ccttctaccc cggcacaggt ttaccacatg 2280

ctgcgtcgtc aggcgttgcg cgggatgcgt cgtccactgg tcgtgatgtc gccgaaatcc 2340

ctgctgcgtc atccgctggc ggtatccagc ctcgaagaac tggcgaacgg caccttcctg 2400

ccagccatcg gtgaaatcga cgagcttgat ccgaagggcg tgaagcgcgt agtgatgtgt 2460

tctggtaagg tttattacga cctgctggaa caacgtcgta agaacaatca acacgatgtc 2520

gccattgtgc gtatcgagca actctacccg ttcccgcata aagcgatgca ggaagtgttg 2580

cagcagtttg ctcacgtcaa ggattttgtc tggtgccagg aagagccgct caaccagggc 2640

gcatggtact gcagccagca tcatttccgt gaagtgattc cgtttggggc ttctctgcgt 2700

tatgcaggcc gcccagcctc cgcctctccg gcggtagggt atatgtccgt tcaccagaaa 2760

cagcaacaag atctggttaa tgacgcgctg aacgtcgaat aa 2802

<210> 5
<211> 453
<212> PRT
<213> Corynebacterium glutamicum

<400> 5
Met Ser Leu Thr Phe Pro Val Ile Asn Pro Ser Asp Gly Ser Thr Ile
1               5                   10                  15

Thr Glu Leu Glu Asn His Asp Ser Thr Gln Trp Met Ser Ala Leu Ser
            20                  25                  30

Asp Ala Val Ala Ala Gly Pro Ser Trp Ala Ala Lys Thr Pro Arg Glu
        35                  40                  45

Arg Ser Val Val Leu Thr Ala Ile Phe Glu Ala Leu Thr Glu Arg Ala
    50                  55                  60

Gln Glu Leu Ala Glu Ile Ile His Leu Glu Ala Gly Lys Ser Val Ala
65                  70                  75                  80

Glu Ala Leu Gly Glu Val Ala Tyr Gly Ala Glu Tyr Phe Arg Trp Phe
                85                  90                  95

Ala Glu Glu Ala Val Arg Leu Pro Gly Arg Tyr Gly Gln Ser Pro Ser
            100                 105                 110

Gly Ile Gly His Ile Ala Val Thr Arg Ala Pro Val Gly Pro Val Leu

        115                 120                 125

Ala Ile Thr Pro Trp Asn Phe Pro Ile Ala Met Ala Thr Arg Lys Ile
    130                 135                 140

Ala Pro Ala Leu Ala Ala Gly Cys Pro Val Leu Val Lys Pro Ala Ser
145                 150                 155                 160

Glu Thr Pro Leu Thr Met Val Lys Val Gly Glu Ile Ile Ala Ser Val
                165                 170                 175

Phe Asp Thr Phe Asn Ile Pro Gln Gly Leu Val Ser Ile Ile Thr Thr
            180                 185                 190

Thr Arg Asp Ala Glu Leu Ser Ala Glu Leu Met Ala Asp Pro Arg Leu
        195                 200                 205

Ala Lys Val Thr Phe Thr Gly Ser Thr Asn Val Gly Arg Ile Leu Val
    210                 215                 220

Arg Gln Ser Ala Asp Arg Leu Leu Arg Thr Ser Met Glu Leu Gly Gly
225                 230                 235                 240

Asn Ala Ala Phe Val Ile Asp Glu Ala Ala Asp Leu Asp Glu Ala Val
                245                 250                 255

Ser Gly Ala Ile Ala Ala Lys Leu Arg Asn Ala Gly Gln Val Cys Ile
            260                 265                 270

Ala Ala Asn Arg Phe Leu Val His Glu Ser Arg Ala Ala Glu Phe Thr
        275                 280                 285

Ser Lys Leu Ala Thr Ala Met Gln Asn Thr Pro Ile Gly Pro Val Ile
    290                 295                 300

Ser Ala Arg Gln Arg Asp Arg Ile Ala Ala Leu Val Asp Glu Ala Ile
305                 310                 315                 320

Thr Asp Gly Ala Arg Leu Ile Ile Gly Gly Glu Val Pro Asp Gly Ser
                325                 330                 335

Gly Phe Phe Tyr Pro Ala Thr Ile Leu Ala Asp Val Pro Ala Gln Ser
            340                 345                 350

Arg Ile Val His Glu Glu Ile Phe Gly Pro Val Ala Thr Ile Ala Thr
        355                 360                 365

Phe Thr Asp Leu Ala Glu Gly Val Ala Gln Ala Asn Ser Thr Glu Phe
    370                 375                 380

Gly Leu Ala Ala Tyr Gly Phe Ser Asn Asn Val Lys Ala Thr Gln Tyr
385                 390                 395                 400

Met Ala Glu His Leu Glu Ala Gly Met Val Gly Ile Asn Arg Gly Ala
                405                 410                 415

Ile Ser Asp Pro Ala Ala Pro Phe Gly Gly Ile Gly Gln Ser Gly Phe
            420                 425                 430

Gly Arg Glu Gly Gly Thr Glu Gly Ile Glu Glu Tyr Leu Ser Val Arg
        435                 440                 445

Tyr Leu Ala Leu Pro

    450

<210>    6
<211>    1362
<212>    DNA
<213>    Corynebacterium glutamicum

<400>    6
gtgtctttga ccttcccagt aatcaacccc agcgatggct ccaccatcac cgagctagaa      60

aaccacgatt ccacccagtg gatgtccgcg ctctctgatg cagttgcagc tggtccttca     120

tgggctgcga aaactccccg cgaaagatcc gtggtactca ccgcaatctt cgaagcactg     180

accgaacgcg cccaagaact tgcagagatc atccacctgg aagctggaaa atccgttgca     240

gaagctcttg gtgaagtcgc ttatggtgca gaatacttcc gttggtttgc ggaagaagca     300

gtgcgcctgc ccggccgcta cggacagtca ccttccggaa tcggtcacat cgccgtcacc     360

cgcgcacccg tgggaccagt gctggcgatc accccatgga atttccccat cgccatggcc     420

acccgcaaaa tcgccccagc cctggccgct ggttgccccg tgttggtgaa acctgcttcc     480

gaaaccccac tgaccatggt caaagtgggg gagatcatcg cctccgtctt tgataccttt     540

aatatcccgc agggcttggt ctcaatcatc accaccactc gagatgcaga gctatcggca     600

gaactcatgg ctgatcctcg cttggctaaa gtcaccttca ctggatcaac caacgtggga     660

cgcatcctgg tccgccaatc cgcggaccga ctgctgcgca cctccatgga actcggcgga     720

aatgcagctt ttgttatcga cgaagccgca gacctcgacg aagccgtatc cggtgccatc     780

gccgcaaaac tccgcaacgc cggccaagta tgcatcgcag ctaaccgttt cttggttcat     840

gaatcccgcg ctgccgaatt cacctcaaag ctggcgacag ccatgcagaa cactcccatt     900

gggccggtga tttctgcccg ccaacgcgac cggatcgcag cactagtgga tgaagccatc     960

accgacggcg cccgcctcat catcggtggg gaggtccccg acggctccgg cttcttctat    1020

ccagccacca tcttggccga tgtccctgca cagtcacgga ttgtgcatga ggaaatcttc    1080

ggacctgtgg ccaccattgc cactttcacc gacttggccg aaggcgttgc acaagcaaat    1140

tccaccgaat tcggcctcgc agcctacgga ttcagcaaca atgtgaaagc aacacagtac    1200

atggcggaac acttggaagc cggaatggtc ggaatcaaca gaggcgccat ctctgaccca    1260

gcagcacctt ttggcggcat cggacaatcc ggcttcggca gagaaggcgg aaccgaagga    1320

atcgaagaat atctctccgt gcgttacctc gctttgccgt ga                        1362

<210>    7
<211>    294
<212>    PRT
<213>    Corynebacterium glutamicum

<400>    7
Met Ser Ile Phe Leu Asn Ser Asp Ser Arg Ile Ile Ile Gln Gly Ile

1                   5                   10                  15

Thr Gly Ser Glu Gly Ser Glu His Ala Arg Arg Ile Leu Ala Ser Gly
                20                  25                  30

Ala Lys Leu Val Gly Gly Thr Asn Pro Arg Lys Ala Gly Gln Thr Ile
            35                  40                  45

Leu Ile Asn Asp Thr Glu Leu Pro Val Phe Gly Thr Val Lys Glu Ala
    50                  55                  60

Met Glu Glu Thr Gly Ala Asp Val Thr Val Ile Phe Val Pro Pro Ala
65                  70                  75                  80

Phe Ala Lys Ala Ala Ile Ile Glu Ala Ile Asp Ala His Ile Pro Leu
                85                  90                  95

Cys Val Ile Ile Thr Glu Gly Ile Pro Val Arg Asp Ala Ser Glu Ala
            100                 105                 110

Trp Ala Tyr Ala Lys Lys Val Gly His Thr Arg Ile Ile Gly Pro Asn
        115                 120                 125

Cys Pro Gly Ile Ile Thr Pro Gly Glu Ser Leu Ala Gly Ile Thr Pro
    130                 135                 140

Ala Asn Ile Ala Gly Ser Gly Pro Ile Gly Leu Ile Ser Lys Ser Gly
145                 150                 155                 160

Thr Leu Thr Tyr Gln Met Met Tyr Glu Leu Ser Asp Ile Gly Ile Ser
                165                 170                 175

Thr Ala Ile Gly Ile Gly Gly Asp Pro Ile Ile Gly Thr Thr His Ile
            180                 185                 190

Asp Ala Leu Glu Ala Phe Glu Ala Asp Pro Glu Thr Lys Ala Ile Val
        195                 200                 205

Met Ile Gly Glu Ile Gly Gly Asp Ala Glu Glu Arg Ala Ala Asp Phe
    210                 215                 220

Ile Ser Lys His Val Thr Lys Pro Val Val Gly Tyr Val Ala Gly Phe
225                 230                 235                 240

Thr Ala Pro Glu Gly Lys Thr Met Gly His Ala Gly Ala Ile Val Thr
                245                 250                 255

Gly Ser Glu Gly Thr Ala Arg Ala Lys Lys His Ala Leu Glu Ala Val
            260                 265                 270

Gly Val Arg Val Gly Thr Thr Pro Ser Glu Thr Ala Lys Leu Met Arg
        275                 280                 285

Glu Val Val Ala Ala Leu
    290

<210>   8
<211>   398
<212>   PRT
<213>   Corynebacterium glutamicum

<400>   8

Met Asp Leu Phe Glu Tyr Gln Ala Arg Asp Leu Phe Glu Thr His Gly
1               5                   10                  15

Val Pro Val Leu Lys Gly Ile Val Ala Ser Thr Pro Glu Ala Ala Arg
            20                  25                  30

Lys Ala Ala Glu Glu Ile Gly Gly Leu Thr Val Val Lys Ala Gln Val
        35                  40                  45

Lys Val Gly Gly Arg Gly Lys Ala Gly Gly Val Arg Val Ala Pro Thr
    50                  55                  60

Ser Ala Gln Ala Phe Asp Ala Ala Asp Ala Ile Leu Gly Met Asp Ile
65                  70                  75                  80

Lys Gly His Thr Val Asn Gln Val Met Val Ala Gln Gly Ala Asp Ile
                85                  90                  95

Ala Glu Glu Tyr Tyr Phe Ser Ile Leu Leu Asp Arg Ala Asn Arg Ser
            100                 105                 110

Tyr Leu Ala Met Cys Ser Val Glu Gly Gly Met Glu Ile Glu Ile Leu
        115                 120                 125

Ala Lys Glu Lys Pro Glu Ala Leu Ala Lys Val Glu Val Asp Pro Leu
    130                 135                 140

Thr Gly Ile Asp Glu Asp Lys Ala Arg Glu Ile Val Thr Ala Ala Gly
145                 150                 155                 160

Phe Glu Thr Glu Val Ala Glu Lys Val Ile Pro Val Leu Ile Lys Ile
                165                 170                 175

Trp Gln Val Tyr Tyr Glu Glu Glu Ala Thr Leu Val Glu Val Asn Pro
            180                 185                 190

Leu Val Leu Thr Asp Asp Gly Asp Val Ile Ala Leu Asp Gly Lys Ile
        195                 200                 205

Thr Leu Asp Asp Asn Ala Asp Phe Arg His Asp Asn Arg Gly Ala Leu
    210                 215                 220

Ala Glu Ser Ala Gly Gly Leu Asp Ile Leu Glu Leu Lys Ala Lys Lys
225                 230                 235                 240

Asn Asp Leu Asn Tyr Val Lys Leu Asp Gly Ser Val Gly Ile Ile Gly
                245                 250                 255

Asn Gly Ala Gly Leu Val Met Ser Thr Leu Asp Ile Val Ala Ala Ala
            260                 265                 270

Gly Glu Arg His Gly Gly Gln Arg Pro Ala Asn Phe Leu Asp Ile Gly
        275                 280                 285

Gly Gly Ala Ser Ala Glu Ser Met Ala Ala Gly Leu Asp Val Ile Leu
    290                 295                 300

Gly Asp Ser Gln Val Arg Ser Val Phe Val Asn Val Phe Gly Gly Ile
305                 310                 315                 320

Thr Ala Cys Asp Val Val Ala Lys Gly Ile Val Gly Ala Leu Asp Val
                325                 330                 335

Leu Gly Asp Gln Ala Thr Lys Pro Leu Val Val Arg Leu Asp Gly Asn
            340                 345                 350

Asn Val Val Glu Gly Arg Arg Ile Leu Ala Glu Tyr Asn His Pro Leu
        355                 360                 365

Val Thr Val Val Glu Gly Met Asp Ala Ala Ala Asp His Ala Ala His
    370                 375                 380

Leu Ala Asn Leu Ala Gln His Gly Gln Phe Ala Thr Ala Asn
385                 390                 395


<210>    9
<211>    314
<212>    PRT
<213>    Corynebacterium glutamicum

<400>    9
Met Lys Glu Thr Val Gly Asn Lys Ile Val Leu Ile Gly Ala Gly Asp
  1               5                  10                  15

Val Gly Val Ala Tyr Ala Tyr Ala Leu Ile Asn Gln Gly Met Ala Asp
             20                  25                  30

His Leu Ala Ile Ile Asp Ile Asp Glu Lys Lys Leu Glu Gly Asn Val
         35                  40                  45

Met Asp Leu Asn His Gly Val Val Trp Ala Asp Ser Arg Thr Arg Val
     50                  55                  60

Thr Lys Gly Thr Tyr Ala Asp Cys Glu Asp Ala Ala Met Val Val Ile
 65                  70                  75                  80

Cys Ala Gly Ala Ala Gln Lys Pro Gly Glu Thr Arg Leu Gln Leu Val
                 85                  90                  95

Asp Lys Asn Val Lys Ile Met Lys Ser Ile Val Gly Asp Val Met Asp
            100                 105                 110

Ser Gly Phe Asp Gly Ile Phe Leu Val Ala Ser Asn Pro Val Asp Ile
        115                 120                 125

Leu Thr Tyr Ala Val Trp Lys Phe Ser Gly Leu Glu Trp Asn Arg Val
    130                 135                 140

Ile Gly Ser Gly Thr Val Leu Asp Ser Ala Arg Phe Arg Tyr Met Leu
145                 150                 155                 160

Gly Glu Leu Tyr Glu Val Ala Pro Ser Ser Val His Ala Tyr Ile Ile
                165                 170                 175

Gly Glu His Gly Asp Thr Glu Leu Pro Val Leu Ser Ser Ala Thr Ile
            180                 185                 190

Ala Gly Val Ser Leu Ser Arg Met Leu Asp Lys Asp Pro Glu Leu Glu
        195                 200                 205

Gly Arg Leu Glu Lys Ile Phe Glu Asp Thr Arg Asp Ala Ala Tyr His
    210                 215                 220

Ile Ile Asp Ala Lys Gly Ser Thr Ser Tyr Gly Ile Gly Met Gly Leu
225                 230                 235                 240

Ala Arg Ile Thr Arg Ala Ile Leu Gln Asn Gln Asp Val Ala Val Pro
                245                 250                 255

Val Ser Ala Leu Leu His Gly Glu Tyr Gly Glu Glu Asp Ile Tyr Ile
            260                 265                 270

Gly Thr Pro Ala Val Val Asn Arg Arg Gly Ile Arg Arg Val Val Glu
        275                 280                 285

Leu Glu Ile Thr Asp His Glu Met Glu Arg Phe Lys His Ser Ala Asn
    290                 295                 300

Thr Leu Arg Glu Ile Gln Lys Gln Phe Phe
305                 310

<210> 10
<211> 579
<212> PRT
<213> Corynebacterium glutamicum

<400> 10
Met Ala His Ser Tyr Ala Glu Gln Leu Ile Asp Thr Leu Glu Ala Gln
1               5                   10                  15

Gly Val Lys Arg Ile Tyr Gly Leu Val Gly Asp Ser Leu Asn Pro Ile
            20                  25                  30

Val Asp Ala Val Arg Gln Ser Asp Ile Glu Trp Val His Val Arg Asn
        35                  40                  45

Glu Glu Ala Ala Ala Phe Ala Ala Gly Ala Glu Ser Leu Ile Thr Gly
    50                  55                  60

Glu Leu Ala Val Cys Ala Ala Ser Cys Gly Pro Gly Asn Thr His Leu
65                  70                  75                  80

Ile Gln Gly Leu Tyr Asp Ser His Arg Asn Gly Ala Lys Val Leu Ala
                85                  90                  95

Ile Ala Ser His Ile Pro Ser Ala Gln Ile Gly Ser Thr Phe Phe Gln
            100                 105                 110

Glu Thr His Pro Glu Ile Leu Phe Lys Glu Cys Ser Gly Tyr Cys Glu
        115                 120                 125

Met Val Asn Gly Gly Glu Gln Gly Glu Arg Ile Leu His His Ala Ile
    130                 135                 140

Gln Ser Thr Met Ala Gly Lys Gly Val Ser Val Val Val Ile Pro Gly
145                 150                 155                 160

Asp Ile Ala Lys Glu Asp Ala Gly Asp Gly Thr Tyr Ser Asn Ser Thr
                165                 170                 175

Ile Ser Ser Gly Thr Pro Val Val Phe Pro Asp Pro Thr Glu Ala Ala
            180                 185                 190

Ala Leu Val Glu Ala Ile Asn Asn Ala Lys Ser Val Thr Leu Phe Cys
        195                 200                 205

Gly Ala Gly Val Lys Asn Ala Arg Ala Gln Val Leu Glu Leu Ala Glu

210                 215                 220

Lys Ile Lys Ser Pro Ile Gly His Ala Leu Gly Gly Lys Gln Tyr Ile
225                 230                 235                 240

Gln His Glu Asn Pro Phe Glu Val Gly Met Ser Gly Leu Leu Gly Tyr
                245                 250                 255

Gly Ala Cys Val Asp Ala Ser Asn Glu Ala Asp Leu Leu Ile Leu Leu
            260                 265                 270

Gly Thr Asp Phe Pro Tyr Ser Asp Phe Leu Pro Lys Asp Asn Val Ala
        275                 280                 285

Gln Val Asp Ile Asn Gly Ala His Ile Gly Arg Arg Thr Thr Val Lys
    290                 295                 300

Tyr Pro Val Thr Gly Asp Val Ala Ala Thr Ile Glu Asn Ile Leu Pro
305                 310                 315                 320

His Val Lys Glu Lys Thr Asp Arg Ser Phe Leu Asp Arg Met Leu Lys
                325                 330                 335

Ala His Glu Arg Lys Leu Ser Ser Val Val Glu Thr Tyr Thr His Asn
            340                 345                 350

Val Glu Lys His Val Pro Ile His Pro Glu Tyr Val Ala Ser Ile Leu
        355                 360                 365

Asn Glu Leu Ala Asp Lys Asp Ala Val Phe Thr Val Asp Thr Gly Met
    370                 375                 380

Cys Asn Val Trp His Ala Arg Tyr Ile Glu Asn Pro Glu Gly Thr Arg
385                 390                 395                 400

Asp Phe Val Gly Ser Phe Arg His Gly Thr Met Ala Asn Ala Leu Pro
                405                 410                 415

His Ala Ile Gly Ala Gln Ser Val Asp Arg Asn Arg Gln Val Ile Ala
            420                 425                 430

Met Cys Gly Asp Gly Gly Leu Gly Met Leu Leu Gly Glu Leu Leu Thr
        435                 440                 445

Val Lys Leu His Gln Leu Pro Leu Lys Ala Val Val Phe Asn Asn Ser
    450                 455                 460

Ser Leu Gly Met Val Lys Leu Glu Met Leu Val Glu Gly Gln Pro Glu
465                 470                 475                 480

Phe Gly Thr Asp His Glu Glu Val Asn Phe Ala Glu Ile Ala Ala Ala
                485                 490                 495

Ala Gly Ile Lys Ser Val Arg Ile Thr Asp Pro Lys Lys Val Arg Glu
            500                 505                 510

Gln Leu Ala Glu Ala Leu Ala Tyr Pro Gly Pro Val Leu Ile Asp Ile
        515                 520                 525

Val Thr Asp Pro Asn Ala Leu Ser Ile Pro Pro Thr Ile Thr Trp Glu
    530                 535                 540

Gln Val Met Gly Phe Ser Lys Ala Ala Thr Arg Thr Val Phe Gly Gly

545                 550                 555                 560

Gly Val Gly Ala Met Ile Asp Leu Ala Arg Ser Asn Ile Arg Asn Ile
                565                 570                 575

Pro Thr Pro

<210>   11
<211>   461
<212>   PRT
<213>   Corynebacterium glutamicum

<400>   11
Met Ser Asp Thr Pro Thr Ser Ala Leu Ile Thr Thr Val Asn Arg Ser
1               5                   10                  15

Phe Asp Gly Phe Asp Leu Glu Glu Val Ala Ala Asp Leu Gly Val Arg
            20                  25                  30

Leu Thr Tyr Leu Pro Asp Glu Glu Leu Glu Val Ser Lys Val Leu Ala
        35                  40                  45

Ala Asp Leu Leu Ala Glu Gly Pro Ala Leu Ile Ile Gly Val Gly Asn
    50                  55                  60

Thr Phe Phe Asp Ala Gln Val Ala Ala Ala Leu Gly Val Pro Val Leu
65                  70                  75                  80

Leu Leu Val Asp Lys Gln Gly Lys His Val Ala Leu Ala Arg Thr Gln
                85                  90                  95

Val Asn Asn Ala Gly Ala Val Val Ala Ala Ala Phe Thr Ala Glu Gln
            100                 105                 110

Glu Pro Met Pro Asp Lys Leu Arg Lys Ala Val Arg Asn His Ser Asn
        115                 120                 125

Leu Glu Pro Val Met Ser Ala Glu Leu Phe Glu Asn Trp Leu Leu Lys
    130                 135                 140

Arg Ala Arg Ala Glu His Ser His Ile Val Leu Pro Glu Gly Asp Asp
145                 150                 155                 160

Asp Arg Ile Leu Met Ala Ala His Gln Leu Leu Asp Gln Asp Ile Cys
                165                 170                 175

Asp Ile Thr Ile Leu Gly Asp Pro Val Lys Ile Lys Glu Arg Ala Thr
            180                 185                 190

Glu Leu Gly Leu His Leu Asn Thr Ala Tyr Leu Val Asn Pro Leu Thr
        195                 200                 205

Asp Pro Arg Leu Glu Glu Phe Ala Glu Gln Phe Ala Glu Leu Arg Lys
    210                 215                 220

Ser Lys Ser Val Thr Ile Asp Glu Ala Arg Glu Ile Met Lys Asp Ile
225                 230                 235                 240

Ser Tyr Phe Gly Thr Met Met Val His Asn Gly Asp Ala Asp Gly Met
                245                 250                 255

Val Ser Gly Ala Ala Asn Thr Thr Ala His Thr Ile Lys Pro Ser Phe
            260                 265                 270

Gln Ile Ile Lys Thr Val Pro Glu Ala Ser Val Val Ser Ser Ile Phe
        275                 280                 285

Leu Met Val Leu Arg Gly Arg Leu Trp Ala Phe Gly Asp Cys Ala Val
    290                 295                 300

Asn Pro Asn Pro Thr Ala Glu Gln Leu Gly Glu Ile Ala Val Val Ser
305                 310                 315                 320

Ala Lys Thr Ala Ala Gln Phe Gly Ile Asp Pro Arg Val Ala Ile Leu
                325                 330                 335

Ser Tyr Ser Thr Gly Asn Ser Gly Gly Gly Ser Asp Val Asp Arg Ala
            340                 345                 350

Ile Asp Ala Leu Ala Glu Ala Arg Arg Leu Asn Pro Glu Leu Cys Val
        355                 360                 365

Asp Gly Pro Leu Gln Phe Asp Ala Ala Val Asp Pro Gly Val Ala Arg
    370                 375                 380

Lys Lys Met Pro Asp Ser Asp Val Ala Gly Gln Ala Asn Val Phe Ile
385                 390                 395                 400

Phe Pro Asp Leu Glu Ala Gly Asn Ile Gly Tyr Lys Thr Ala Gln Arg
                405                 410                 415

Thr Gly His Ala Leu Ala Val Gly Pro Ile Leu Gln Gly Leu Asn Lys
            420                 425                 430

Pro Val Asn Asp Leu Ser Arg Gly Ala Thr Val Pro Asp Ile Val Asn
        435                 440                 445

Thr Val Ala Ile Thr Ala Ile Gln Ala Gly Gly Arg Ser
    450                 455                 460

<210> 12
<211> 397
<212> PRT
<213> Corynebacterium glutamicum

<400> 12
Met Ala Leu Ala Leu Val Leu Asn Ser Gly Ser Ser Ser Ile Lys Phe
1               5                   10                  15

Gln Leu Val Asn Pro Glu Asn Ser Ala Ile Asp Glu Pro Tyr Val Ser
            20                  25                  30

Gly Leu Val Glu Gln Ile Gly Glu Pro Asn Gly Arg Ile Val Leu Lys
        35                  40                  45

Ile Glu Gly Glu Lys Tyr Thr Leu Glu Thr Pro Ile Ala Asp His Ser
    50                  55                  60

Glu Gly Leu Asn Leu Ala Phe Asp Leu Met Asp Gln His Asn Cys Gly
65                  70                  75                  80

Pro Ser Gln Leu Glu Ile Thr Ala Val Gly His Arg Val Val His Gly
                85                  90                  95

Gly Ile Leu Phe Ser Ala Pro Glu Leu Ile Thr Asp Glu Ile Val Glu
            100                 105                 110

Met Ile Arg Asp Leu Ile Pro Leu Ala Pro Leu His Asn Pro Ala Asn
        115                 120                 125

Val Asp Gly Ile Asp Val Ala Arg Lys Ile Leu Pro Asp Val Pro His
    130                 135                 140

Val Ala Val Phe Asp Thr Gly Phe Phe His Ser Leu Pro Pro Ala Ala
145                 150                 155                 160

Ala Leu Tyr Ala Ile Asn Lys Asp Val Ala Ala Glu His Gly Ile Arg
                165                 170                 175

Arg Tyr Gly Phe His Gly Thr Ser His Glu Phe Val Ser Lys Arg Val
            180                 185                 190

Val Glu Ile Leu Glu Lys Pro Thr Glu Asp Ile Asn Thr Ile Thr Phe
        195                 200                 205

His Leu Gly Asn Gly Ala Ser Met Ala Ala Val Gln Gly Gly Arg Ala
    210                 215                 220

Val Asp Thr Ser Met Gly Met Thr Pro Leu Ala Gly Leu Val Met Gly
225                 230                 235                 240

Thr Arg Ser Gly Asp Ile Asp Pro Gly Ile Val Phe His Leu Ser Arg
                245                 250                 255

Thr Ala Gly Met Ser Ile Asp Glu Ile Asp Asn Leu Leu Asn Lys Lys
            260                 265                 270

Ser Gly Val Lys Gly Leu Ser Gly Val Asn Asp Phe Arg Glu Leu Arg
        275                 280                 285

Glu Met Ile Asp Asn Asn Asp Gln Asp Ala Trp Ser Ala Tyr Asn Ile
    290                 295                 300

Tyr Ile His Gln Leu Arg Arg Tyr Leu Gly Ser Tyr Met Val Ala Leu
305                 310                 315                 320

Gly Arg Val Asp Thr Ile Val Phe Thr Ala Gly Val Gly Glu Asn Ala
                325                 330                 335

Gln Phe Val Arg Glu Asp Ala Leu Ala Gly Leu Glu Met Tyr Gly Ile
            340                 345                 350

Glu Ile Asp Pro Glu Arg Asn Ala Leu Pro Asn Asp Gly Pro Arg Leu
        355                 360                 365

Ile Ser Thr Asp Ala Ser Lys Val Lys Val Phe Val Ile Pro Thr Asn
    370                 375                 380

Glu Glu Leu Ala Ile Ala Arg Tyr Ala Val Lys Phe Ala
385                 390                 395

<210>   13
<211>   250

<212>    PRT
<213>    Corynebacterium glutamicum

<400>    13
Met Ser His Met Ile Asn Lys Ser Ile Ser Ser Thr Ala Glu Ala Val
1               5                   10                  15

Ala Asp Ile Pro Asp Gly Ala Ser Ile Ala Val Gly Gly Phe Gly Leu
            20                  25                  30

Val Gly Ile Pro Thr Ala Leu Ile Leu Ala Leu Arg Glu Gln Gly Ala
        35                  40                  45

Gly Asp Leu Thr Ile Ile Ser Asn Asn Leu Gly Thr Asp Gly Phe Gly
    50                  55                  60

Leu Gly Leu Leu Leu Leu Asp Lys Lys Ile Ser Lys Ser Ile Gly Ser
65                  70                  75                  80

Tyr Leu Gly Ser Asn Lys Glu Tyr Ala Arg Gln Tyr Leu Glu Gly Glu
                85                  90                  95

Leu Thr Val Glu Phe Thr Pro Gln Gly Thr Leu Ala Glu Arg Leu Arg
            100                 105                 110

Ala Gly Gly Ala Gly Ile Pro Ala Phe Tyr Thr Thr Ala Gly Val Gly
        115                 120                 125

Thr Gln Val Ala Glu Gly Gly Leu Pro Gln Arg Tyr Asn Thr Asp Gly
    130                 135                 140

Thr Val Ala Val Val Ser Gln Pro Lys Glu Thr Arg Glu Phe Asn Gly
145                 150                 155                 160

Gln Leu Tyr Val Met Glu Glu Gly Ile Arg Ala Asp Tyr Ala Leu Val
                165                 170                 175

His Ala His Lys Ala Asp Arg Phe Gly Asn Leu Val Phe Arg Lys Thr
            180                 185                 190

Ala Gln Asn Phe Asn Pro Asp Ala Ala Met Ser Gly Lys Ile Thr Ile
        195                 200                 205

Ala Gln Val Glu His Phe Val Asp Glu Leu His Pro Asp Glu Ile Asp
    210                 215                 220

Leu Pro Gly Ile Tyr Val Asn Arg Val Val His Val Gly Pro Gln Glu
225                 230                 235                 240

Thr Gly Ile Glu Asn Arg Thr Val Ser Asn
                245                 250


<210>    14
<211>    1140
<212>    PRT
<213>    Corynebacterium glutamicum

<400>    14
Met Ser Thr His Thr Ser Ser Thr Leu Pro Ala Phe Lys Lys Ile Leu
1               5                   10                  15

Val Ala Asn Arg Gly Glu Ile Ala Val Arg Ala Phe Arg Ala Ala Leu

20                  25                  30

Glu Thr Gly Ala Ala Thr Val Ala Ile Tyr Pro Arg Glu Asp Arg Gly
        35                  40                  45

Ser Phe His Arg Ser Phe Ala Ser Glu Ala Val Arg Ile Gly Thr Glu
    50                  55                  60

Gly Ser Pro Val Lys Ala Tyr Leu Asp Ile Asp Glu Ile Ile Gly Ala
65                  70                  75                  80

Ala Lys Lys Val Lys Ala Asp Ala Ile Tyr Pro Gly Tyr Gly Phe Leu
                85                  90                  95

Ser Glu Asn Ala Gln Leu Ala Arg Glu Cys Ala Glu Asn Gly Ile Thr
            100                 105                 110

Phe Ile Gly Pro Thr Pro Glu Val Leu Asp Leu Thr Gly Asp Lys Ser
        115                 120                 125

Arg Ala Val Thr Ala Ala Lys Lys Ala Gly Leu Pro Val Leu Ala Glu
    130                 135                 140

Ser Thr Pro Ser Lys Asn Ile Asp Glu Ile Val Lys Ser Ala Glu Gly
145                 150                 155                 160

Gln Thr Tyr Pro Ile Phe Val Lys Ala Val Ala Gly Gly Gly Gly Arg
                165                 170                 175

Gly Met Arg Phe Val Ala Ser Pro Asp Glu Leu Arg Lys Leu Ala Thr
            180                 185                 190

Glu Ala Ser Arg Glu Ala Glu Ala Ala Phe Gly Asp Gly Ala Val Tyr
        195                 200                 205

Val Glu Arg Ala Val Ile Asn Pro Gln His Ile Glu Val Gln Ile Leu
    210                 215                 220

Gly Asp His Thr Gly Glu Val Val His Leu Tyr Glu Arg Asp Cys Ser
225                 230                 235                 240

Leu Gln Arg Arg His Gln Lys Val Val Glu Ile Ala Pro Ala Gln His
                245                 250                 255

Leu Asp Pro Glu Leu Arg Asp Arg Ile Cys Ala Asp Ala Val Lys Phe
            260                 265                 270

Cys Arg Ser Ile Gly Tyr Gln Gly Ala Gly Thr Val Glu Phe Leu Val
        275                 280                 285

Asp Glu Lys Gly Asn His Val Phe Ile Glu Met Asn Pro Arg Ile Gln
    290                 295                 300

Val Glu His Thr Val Thr Glu Glu Val Thr Glu Val Asp Leu Val Lys
305                 310                 315                 320

Ala Gln Met Arg Leu Ala Ala Gly Ala Thr Leu Lys Glu Leu Gly Leu
                325                 330                 335

Thr Gln Asp Lys Ile Lys Thr His Gly Ala Ala Leu Gln Cys Arg Ile
            340                 345                 350

Thr Thr Glu Asp Pro Asn Asn Gly Phe Arg Pro Asp Thr Gly Thr Ile

        355                 360                 365

Thr Ala Tyr Arg Ser Pro Gly Gly Ala Gly Val Arg Leu Asp Gly Ala
    370                 375                 380

Ala Gln Leu Gly Gly Glu Ile Thr Ala His Phe Asp Ser Met Leu Val
385                 390                 395                 400

Lys Met Thr Cys Arg Gly Ser Asp Phe Glu Thr Ala Val Ala Arg Ala
                405                 410                 415

Gln Arg Ala Leu Ala Glu Phe Thr Val Ser Gly Val Ala Thr Asn Ile
            420                 425                 430

Gly Phe Leu Arg Ala Leu Leu Arg Glu Glu Asp Phe Thr Ser Lys Arg
        435                 440                 445

Ile Ala Thr Gly Phe Ile Ala Asp His Pro His Leu Leu Gln Ala Pro
    450                 455                 460

Pro Ala Asp Asp Glu Gln Gly Arg Ile Leu Asp Tyr Leu Ala Asp Val
465                 470                 475                 480

Thr Val Asn Lys Pro His Gly Val Arg Pro Lys Asp Val Ala Ala Pro
                485                 490                 495

Ile Asp Lys Leu Pro Asn Ile Lys Asp Leu Pro Leu Pro Arg Gly Ser
            500                 505                 510

Arg Asp Arg Leu Lys Gln Leu Gly Pro Ala Ala Phe Ala Arg Asp Leu
        515                 520                 525

Arg Glu Gln Asp Ala Leu Ala Val Thr Asp Thr Thr Phe Arg Asp Ala
    530                 535                 540

His Gln Ser Leu Leu Ala Thr Arg Val Arg Ser Phe Ala Leu Lys Pro
545                 550                 555                 560

Ala Ala Glu Ala Val Ala Lys Leu Thr Pro Glu Leu Leu Ser Val Glu
                565                 570                 575

Ala Trp Gly Gly Ala Thr Tyr Asp Val Ala Met Arg Phe Leu Phe Glu
            580                 585                 590

Asp Pro Trp Asp Arg Leu Asp Glu Leu Arg Glu Ala Met Pro Asn Val
        595                 600                 605

Asn Ile Gln Met Leu Leu Arg Gly Arg Asn Thr Val Gly Tyr Thr Pro
    610                 615                 620

Tyr Pro Asp Ser Val Cys Arg Ala Phe Val Lys Glu Ala Ala Ser Ser
625                 630                 635                 640

Gly Val Asp Ile Phe Arg Ile Phe Asp Ala Leu Asn Asp Val Ser Gln
                645                 650                 655

Met Arg Pro Ala Ile Asp Ala Val Leu Glu Thr Asn Thr Ala Val Ala
            660                 665                 670

Glu Val Ala Met Ala Tyr Ser Gly Asp Leu Ser Asp Pro Asn Glu Lys
        675                 680                 685

Leu Tyr Thr Leu Asp Tyr Tyr Leu Lys Met Ala Glu Glu Ile Val Lys

690                 695                 700

Ser Gly Ala His Ile Leu Ala Ile Lys Asp Met Ala Gly Leu Leu Arg
705                 710                 715                 720

Pro Ala Ala Val Thr Lys Leu Val Thr Ala Leu Arg Arg Glu Phe Asp
                725                 730                 735

Leu Pro Val His Val His Thr His Asp Thr Ala Gly Gly Gln Leu Ala
            740                 745                 750

Thr Tyr Phe Ala Ala Ala Gln Ala Gly Ala Asp Ala Val Asp Gly Ala
        755                 760                 765

Ser Ala Pro Leu Ser Gly Thr Thr Ser Gln Pro Ser Leu Ser Ala Ile
    770                 775                 780

Val Ala Ala Phe Ala His Thr Arg Arg Asp Thr Gly Leu Ser Leu Glu
785                 790                 795                 800

Ala Val Ser Asp Leu Glu Pro Tyr Trp Glu Ala Val Arg Gly Leu Tyr
                805                 810                 815

Leu Pro Phe Glu Ser Gly Thr Pro Gly Pro Thr Gly Arg Val Tyr Arg
            820                 825                 830

His Glu Ile Pro Gly Gly Gln Leu Ser Asn Leu Arg Ala Gln Ala Thr
        835                 840                 845

Ala Leu Gly Leu Ala Asp Arg Phe Glu Leu Ile Glu Asp Asn Tyr Ala
    850                 855                 860

Ala Val Asn Glu Met Leu Gly Arg Pro Thr Lys Val Thr Pro Ser Ser
865                 870                 875                 880

Lys Val Val Gly Asp Leu Ala Leu His Leu Val Gly Ala Gly Val Asp
                885                 890                 895

Pro Ala Asp Phe Ala Ala Asp Pro Gln Lys Tyr Asp Ile Pro Asp Ser
            900                 905                 910

Val Ile Ala Phe Leu Arg Gly Glu Leu Gly Asn Pro Pro Gly Gly Trp
        915                 920                 925

Pro Glu Pro Leu Arg Thr Arg Ala Leu Glu Gly Arg Ser Glu Gly Lys
    930                 935                 940

Ala Pro Leu Thr Glu Val Pro Glu Glu Glu Gln Ala His Leu Asp Ala
945                 950                 955                 960

Asp Asp Ser Lys Glu Arg Arg Asn Ser Leu Asn Arg Leu Leu Phe Pro
                965                 970                 975

Lys Pro Thr Glu Glu Phe Leu Glu His Arg Arg Arg Phe Gly Asn Thr
            980                 985                 990

Ser Ala Leu Asp Asp Arg Glu Phe Phe Tyr Gly Leu Val Glu Gly Arg
        995                 1000                1005

Glu Thr Leu Ile Arg Leu Pro Asp Val Arg Thr Pro Leu Leu Val Arg
    1010                1015                1020

Leu Asp Ala Ile Ser Glu Pro Asp Asp Lys Gly Met Arg Asn Val Val

1025                1030                1035                1040

Ala Asn Val Asn Gly Gln Ile Arg Pro Met Arg Val Arg Asp Arg Ser
            1045                1050                1055

Val Glu Ser Val Thr Ala Thr Ala Glu Lys Ala Asp Ser Ser Asn Lys
        1060                1065                1070

Gly His Val Ala Ala Pro Phe Ala Gly Val Val Thr Val Thr Val Ala
    1075                1080                1085

Glu Gly Asp Glu Val Lys Ala Gly Asp Ala Val Ala Ile Ile Glu Ala
1090                1095                1100

Met Lys Met Glu Ala Thr Ile Thr Ala Ser Val Asp Gly Lys Ile Asp
1105                1110                1115                1120

Arg Val Val Val Pro Ala Ala Thr Lys Val Glu Gly Gly Asp Leu Ile
            1125                1130                1135

Val Val Val Ser
        1140


<210>    15
<211>    43
<212>    DNA
<213>    Artificial Sequence

<220>
<223>    ldhA_5'_HindIII


<400>    15
catgattacg ccaagcttga gagcccacca cattgcgatt tcc                          43


<210>    16
<211>    42
<212>    DNA
<213>    Artificial Sequence

<220>
<223>    ldhA_up_3'_XhoI


<400>    16
tcgaaactcg agtttcgatc ccacttcctg atttccctaa cc                           42


<210>    17
<211>    39
<212>    DNA
<213>    Artificial Sequence

<220>
<223>    ldhA_dn_5'_XhoI


<400>    17
tcgaaactcg agtaaatctt tggcgcctag ttggcgacg                               39

<210>	18
<211>	46
<212>	DNA
<213>	Artificial Sequence

<220>
<223>	ldhA_3'_EcoRI


<400>	18
acgacggcca gtgaattcga cgacatctga gggtggataa agtggg	46


<210>	19
<211>	42
<212>	DNA
<213>	Artificial Sequence

<220>
<223>	poxB 5' H3


<400>	19
catgattacg ccaagctttc agcgtgggtc gggttctttg ag	42


<210>	20
<211>	32
<212>	DNA
<213>	Artificial Sequence

<220>
<223>	DpoxB_up 3'


<400>	20
aatcatcatc tgaactcctc aacgttatgg ct	32


<210>	21
<211>	37
<212>	DNA
<213>	Artificial Sequence

<220>
<223>	DpoxB_dn 5'


<400>	21
ggagttcaga tgatgattga tacacctgct gttctca	37


<210>	22
<211>	44
<212>	DNA
<213>	Artificial Sequence

<220>
<223>	poxB 3' E1


<400>	22
acgacggcca gtgaattcat gtcccgaatc cacttcaatc agag	44

<210>	23
<211>	44
<212>	DNA
<213>	Artificial Sequence

<220>
<223>	pta 5′ H3


<400>	23
catgattacg ccaagcttcc ctccatgata cgtggtaagt gcag	44


<210>	24
<211>	43
<212>	DNA
<213>	Artificial Sequence

<220>
<223>	Dpta_up_R1 3′


<400>	24
gttccctgtt aatgtaacca gctgaggtcg gtgtgtcaga cat	43


<210>	25
<211>	48
<212>	DNA
<213>	Artificial Sequence

<220>
<223>	DackA_dn_R1 5′


<400>	25
ttacattaac agggaaccgg aagagttagc tatcgctagg tacgcggt	48


<210>	26
<211>	40
<212>	DNA
<213>	Artificial Sequence

<220>
<223>	ackA 3′ Xb


<400>	26
acccggggat cctctagagg gctgatgtga tttctgcggg	40


<210>	27
<211>	41
<212>	DNA
<213>	Artificial Sequence

<220>
<223>	actA 5′ Xb

<400>    27
ggtggcggcc gctctagagg tctgagcttt attcctgggc t                                41

<210>    28
<211>    36
<212>    DNA
<213>    Artificial Sequence

<220>
<223>    DactA_up_R4 3'

<400>    28
tctggataga agcatctaag ccagcgccgg tgaagc                                      36

<210>    29
<211>    46
<212>    DNA
<213>    Artificial Sequence

<220>
<223>    DactA_dn_R4 5'

<400>    29
agatgcttct atccagagct ccggtgacaa caagtacatg cagacc                           46

<210>    30
<211>    39
<212>    DNA
<213>    Artificial Sequence

<220>
<223>    actA 3' H3

<400>    30
gacggtatcg ataagcttcg tacgatgctt gagcggtat                                   39

<210>    31
<211>    19
<212>    DNA
<213>    Artificial Sequence

<220>
<223>    poxB_up_for

<400>    31
ggctgaaacc aaaccagac                                                         19

<210>    32
<211>    22
<212>    DNA
<213>    Artificial Sequence

<220>
<223>    poxB_dn_rev

<400>    32
ctgcatgatc ggttagatac ag                                                22

<210>    33
<211>    18
<212>    DNA
<213>    Artificial Sequence

<220>
<223>    pta_up_for

<400>    33
gcgtggaatt gagatcgg                                                     18

<210>    34
<211>    18
<212>    DNA
<213>    Artificial Sequence

<220>
<223>    ackA_dn_rev

<400>    34
cagagcgatt tgtggtgg                                                     18

<210>    35
<211>    20
<212>    DNA
<213>    Artificial Sequence

<220>
<223>    actA_up_for

<400>    35
tgaagcaatg gtgtgaactg                                                   20

<210>    36
<211>    19
<212>    DNA
<213>    Artificial Sequence

<220>
<223>    actA_dn_rev

<400>    36
gctaccaaac actagcctg                                                    19

<210>    37
<211>    25
<212>    DNA
<213>    Artificial Sequence

<220>
<223>    pyc-F1


<400>    37
gctctagatt gagcacaccg tgact                                              25


<210>    38
<211>    19
<212>    DNA
<213>    Artificial Sequence

<220>
<223>    pyc-R1


<400>    38
ctgaaggagg tgcgagtga                                                     19


<210>    39
<211>    19
<212>    DNA
<213>    Artificial Sequence

<220>
<223>    pyc-F2


<400>    39
tcactcgcac ctccttcag                                                     19


<210>    40
<211>    31
<212>    DNA
<213>    Artificial Sequence

<220>
<223>    pyc-R2


<400>    40
gctctagaga agcagcatct gaatgtttac a                                       31


<210>    41
<211>    46
<212>    DNA
<213>    Artificial Sequence

<220>
<223>    MD-616


<400>    41
aaagtgtaaa gcctgggaac aacaagaccc atcatagttt gccccc                       46


<210>    42
<211>    36
<212>    DNA

<213>    Artificial Sequence

<220>
<223>    MD-618


<400>    42
gttcttctaa tcagaattgg ttaattggtt gtaaca                                36


<210>    43
<211>    40
<212>    DNA
<213>    Artificial Sequence

<220>
<223>    MD-615


<400>    43
gcgtaatagc gaagaggggc gtttttccat aggctccgcc                            40


<210>    44
<211>    40
<212>    DNA
<213>    Artificial Sequence

<220>
<223>    MD-617


<400>    44
gttcaatcat aacacccctt gtattactgt ttatgtaagc                            40


<210>    45
<211>    31
<212>    DNA
<213>    Artificial Sequence

<220>
<223>    MD-619


<400>    45
gggtgttatg attgaacaag atggattgca c                                     31


<210>    46
<211>    39
<212>    DNA
<213>    Artificial Sequence

<220>
<223>    MD-620


<400>    46
attctgatta gaagaactcg tcaagaaggc gatagaagg                             39


<210>    47

<211>    17
<212>    DNA
<213>    Artificial Sequence

<220>
<223>    LacZa-NR


<400>    47
cctcttcgct attacgc                                                      17


<210>    48
<211>    21
<212>    DNA
<213>    Artificial Sequence

<220>
<223>    MD-404


<400>    48
cccaggcttt acactttatg c                                                 21


<210>    49
<211>    47
<212>    DNA
<213>    Artificial Sequence

<220>
<223>    MD-627


<400>    49
gccaccgcgg tggagctcat ttagcggatg attctcgttc aacttcg                     47


<210>    50
<211>    32
<212>    DNA
<213>    Artificial Sequence

<220>
<223>    MD-628


<400>    50
ttttatttgc aaaaacggcc gaaaccatcc ct                                     32


<210>    51
<211>    40
<212>    DNA
<213>    Artificial Sequence

<220>
<223>    MD-629


<400>    51
ccgtttttgc aaataaaacg aaaggctcag tcgaaagact                             40

<210>    52
<211>    43
<212>    DNA
<213>    Artificial Sequence

<220>
<223>    MD-630


<400>    52
gaacaaaagc tggagctacc gtatctgtgg ggggatggct tgt                    43


<210>    53
<211>    36
<212>    DNA
<213>    Artificial Sequence

<220>
<223>    Tuf-F


<400>    53
ctatagggcg aattgggatc acagtaggcg cgtagg                            36


<210>    54
<211>    56
<212>    DNA
<213>    Artificial Sequence

<220>
<223>    Tuf-R


<400>    54
gacctcgagg gggggcccgg taccggttgt cctcctttgg gtggctacga ctttcg      56


<210>    55
<211>    26
<212>    DNA
<213>    Artificial Sequence

<220>
<223>    sucA-F


<400>    55
cccaagctta tgcagaacag cgcttt                                       26


<210>    56
<211>    26
<212>    DNA
<213>    Artificial Sequence

<220>
<223>    sucA-R


<400>    56

cccaagcttt tattcgacgt tcagcg                                          26

<210>  57
<211>  47
<212>  DNA
<213>  Artificial Sequence

<220>
<223>  gabD2_RBS-F

<400>  57
tagttctaga ctcgataaga gaggacaacg gtgtctttga ccttccc                   47

<210>  58
<211>  24
<212>  DNA
<213>  Artificial Sequence

<220>
<223>  gabD2-R

<400>  58
cgctctagat cacggcaaag cgag                                            24

<210>  59
<211>  19
<212>  DNA
<213>  Artificial Sequence

<220>
<223>  SucA-RTF

<400>  59
gacaccaatg tgaagcagg                                                  19

<210>  60
<211>  18
<212>  DNA
<213>  Artificial Sequence

<220>
<223>  SucA-RTR

<400>  60
tcccagcgga tcgagatt                                                   18

<210>  61
<211>  20
<212>  DNA
<213>  Artificial Sequence

<220>
<223>  gabD2-RTF

<400>    61
ccttcccagt aatcaacccc                                                    20


<210>    62
<211>    20
<212>    DNA
<213>    Artificial Sequence

<220>
<223>    gabD2-RTR


<400>    62
caccataagc gacttcacca                                                    20


<210>    63
<211>    490
<212>    PRT
<213>    E.coli gabD1

<400>    63
Met Thr Ile Asn Val Ser Glu Leu Leu Ala Lys Val Pro Thr Gly Leu
1               5                   10                  15

Leu Ile Gly Asp Ser Trp Val Glu Ala Ser Asp Gly Gly Thr Phe Asp
            20                  25                  30

Val Glu Asn Pro Ala Thr Gly Glu Thr Ile Ala Thr Leu Ala Ser Ala
        35                  40                  45

Thr Ser Glu Asp Ala Leu Ala Ala Leu Asp Ala Ala Cys Ala Val Gln
    50                  55                  60

Ala Glu Trp Ala Arg Met Pro Ala Arg Glu Arg Ser Asn Ile Leu Arg
65                  70                  75                  80

Arg Gly Phe Glu Leu Val Ala Glu Arg Ala Glu Glu Phe Ala Thr Leu
                85                  90                  95

Met Thr Leu Glu Met Gly Lys Pro Leu Ala Glu Ala Arg Gly Glu Val
            100                 105                 110

Thr Tyr Gly Asn Glu Phe Leu Arg Trp Phe Ser Glu Glu Ala Val Arg
        115                 120                 125

Leu Tyr Gly Arg Tyr Gly Thr Thr Pro Glu Gly Asn Leu Arg Met Leu
    130                 135                 140

Thr Ala Leu Lys Pro Val Gly Pro Cys Leu Leu Ile Thr Pro Trp Asn
145                 150                 155                 160

Phe Pro Leu Ala Met Ala Thr Arg Lys Val Ala Pro Ala Ile Ala Ala
                165                 170                 175

Gly Cys Val Met Val Leu Lys Pro Ala Arg Leu Thr Pro Leu Thr Ser
            180                 185                 190

Gln Tyr Phe Ala Gln Thr Met Leu Asp Ala Gly Leu Pro Ala Gly Val
        195                 200                 205

Leu Asn Val Val Ser Gly Ala Ser Ala Ser Ala Ile Ser Asn Pro Ile
    210                 215                 220

Met Glu Asp Asp Arg Leu Arg Lys Val Ser Phe Thr Gly Ser Thr Pro
225                 230                 235                 240

Val Gly Gln Gln Leu Leu Lys Lys Ala Ala Asp Lys Val Leu Arg Thr
                245                 250                 255

Ser Met Glu Leu Gly Gly Asn Ala Pro Phe Ile Val Phe Glu Asp Ala
            260                 265                 270

Asp Leu Asp Leu Ala Ile Glu Gly Ala Met Gly Ala Lys Met Arg Asn
        275                 280                 285

Ile Gly Glu Ala Cys Thr Ala Ala Asn Arg Phe Leu Val His Glu Ser
    290                 295                 300

Val Ala Asp Glu Phe Gly Arg Arg Phe Ala Ala Arg Leu Glu Glu Gln
305                 310                 315                 320

Val Leu Gly Asn Gly Leu Asp Glu Gly Val Thr Val Gly Pro Leu Val
                325                 330                 335

Glu Glu Lys Ala Arg Asp Ser Val Ala Ser Leu Val Asp Ala Ala Val
            340                 345                 350

Ala Glu Gly Ala Thr Val Leu Thr Gly Gly Lys Ala Gly Thr Gly Ala
        355                 360                 365

Gly Tyr Phe Tyr Glu Pro Thr Val Leu Thr Gly Val Ser Thr Asp Ala
    370                 375                 380

Ala Ile Leu Asn Glu Glu Ile Phe Gly Pro Val Ala Pro Ile Val Thr
385                 390                 395                 400

Phe Gln Thr Glu Glu Glu Ala Leu Arg Leu Ala Asn Ser Thr Glu Tyr
                405                 410                 415

Gly Leu Ala Ser Tyr Val Phe Thr Gln Asp Thr Ser Arg Ile Phe Arg
            420                 425                 430

Val Ser Asp Gly Leu Glu Phe Gly Leu Val Gly Val Asn Ser Gly Val
        435                 440                 445

Ile Ser Asn Ala Ala Ala Pro Phe Gly Gly Val Lys Gln Ser Gly Met
    450                 455                 460

Gly Arg Glu Gly Gly Leu Glu Gly Ile Glu Glu Tyr Thr Ser Val Gln
465                 470                 475                 480

Tyr Ile Gly Ile Arg Asp Pro Tyr Ala Gly
                485                 490


<210>    64
<211>    521
<212>    PRT
<213>    E.coli gabD3

<400>    64
Met Ile Lys Arg Leu Pro Leu Gly Pro Leu Pro Lys Glu Leu His Gln
1               5                   10                  15

Thr Leu Leu Asp Leu Thr Ala Asn Ala Gln Asp Ala Ala Lys Val Glu
            20                  25                  30

Val Ile Ala Pro Phe Thr Gly Glu Thr Leu Gly Phe Val Phe Asp Gly
        35                  40                  45

Asp Glu Gln Asp Val Glu His Ala Phe Ala Leu Ser Arg Ala Ala Gln
    50                  55                  60

Lys Lys Trp Val His Thr Thr Ala Val Glu Arg Lys Lys Ile Phe Leu
65                  70                  75                  80

Lys Phe His Asp Leu Val Leu Lys Asn Arg Glu Leu Leu Met Asp Ile
                85                  90                  95

Val Gln Leu Glu Thr Gly Lys Asn Arg Ala Ser Ala Ala Asp Glu Val
            100                 105                 110

Leu Asp Val Ala Ile Thr Thr Arg Phe Tyr Ala Asn Asn Ala Gly Lys
        115                 120                 125

Phe Leu Asn Asp Lys Lys Arg Pro Gly Ala Leu Pro Ile Ile Thr Lys
    130                 135                 140

Asn Thr Gln Gln Tyr Val Pro Lys Gly Val Val Gly Gln Ile Thr Pro
145                 150                 155                 160

Trp Asn Tyr Pro Leu Thr Leu Gly Val Ser Asp Ala Val Pro Ala Leu
                165                 170                 175

Leu Ala Gly Asn Ala Val Val Ala Lys Pro Asp Leu Ala Thr Pro Phe
            180                 185                 190

Ser Cys Leu Ile Met Val His Leu Leu Ile Glu Ala Gly Leu Pro Arg
        195                 200                 205

Asp Leu Met Gln Val Val Thr Gly Pro Gly Asp Ile Val Gly Gly Ala
    210                 215                 220

Ile Ala Ala Gln Cys Asp Phe Leu Met Phe Thr Gly Ser Thr Ala Thr
225                 230                 235                 240

Gly Arg Ile Leu Gly Arg Thr Met Gly Glu Arg Leu Val Gly Phe Ser
                245                 250                 255

Ala Glu Leu Gly Gly Lys Asn Pro Leu Ile Val Ala Lys Asp Ala Asp
            260                 265                 270

Leu Asp Lys Val Glu Ala Glu Leu Pro Gln Ala Cys Phe Ser Asn Ser
        275                 280                 285

Gly Gln Leu Cys Val Ser Thr Glu Arg Ile Tyr Val Glu Glu Asp Val
    290                 295                 300

Tyr Glu Glu Val Ile Ala Arg Phe Ser Lys Ala Ala Lys Ala Met Ser
305                 310                 315                 320

Ile Gly Ala Gly Phe Glu Trp Lys Tyr Glu Met Gly Ser Leu Ile Asn
                325                 330                 335

Gln Ala Gln Leu Asp Arg Val Ser Thr Phe Val Asp Gln Ala Lys Ala
            340                 345                 350

Ala Gly Ala Thr Val Leu Cys Gly Gly Lys Ser Arg Pro Asp Ile Gly
        355                 360                 365

Pro Phe Phe Tyr Glu Pro Thr Val Leu Ala Asp Val Pro Glu Gly Thr
    370                 375                 380

Pro Leu Leu Thr Glu Glu Val Phe Gly Pro Val Val Phe Ile Glu Lys
385                 390                 395                 400

Val Ala Thr Leu Glu Glu Ala Val Asp Lys Ala Asn Gly Thr Pro Tyr
                405                 410                 415

Gly Leu Asn Ala Ser Val Phe Gly Ser Ser Glu Thr Gly Asn Leu Val
            420                 425                 430

Ala Gly Gln Leu Glu Ala Gly Gly Ile Gly Ile Asn Asp Gly Tyr Ala
        435                 440                 445

Ala Thr Trp Ala Ser Val Ser Thr Pro Leu Gly Gly Met Lys Gln Ser
    450                 455                 460

Gly Leu Gly His Arg His Gly Ala Glu Gly Ile Thr Lys Tyr Ala Glu
465                 470                 475                 480

Ile Arg Asn Ile Ala Glu Gln Arg Trp Met Ser Met Arg Gly Pro Ala
                485                 490                 495

Lys Met Pro Arg Lys Val Tyr Ser Asp Thr Val Ala Thr Ala Leu Lys
            500                 505                 510

Leu Gly Lys Ile Phe Lys Val Leu Pro
        515                 520


<210>    65
<211>    1473
<212>    DNA
<213>    E.coli gabD1

<400>    65
atgactatta atgtctccga actacttgcc aaagtcccca cgggtctact gattggtgat      60

tcctgggtgg aagcatccga cggcggtact ttcgatgtgg aaaacccagc gacgggtgaa     120

acaatcgcaa cgctcgcgtc tgctacttcc gaggatgcac tggctgctct tgatgctgca     180

tgcgctgttc aggccgagtg ggctaggatg ccagcgcgcg agcgttctaa tattttacgc     240

cgcggttttg agctcgtagc agaacgtgca gaagagttcg ccaccctcat gaccttggaa     300

atgggcaagc ctttggctga agctcgcggc gaagtcacct acggcaacga attcctgcgc     360

tggttctctg aggaagcagt tcgtctgtat ggccgttacg gaaccacacc agaaggcaac     420

ttgcggatgc tgaccgccct caagccagtt ggcccgtgcc tcctgatcac cccatggaac     480

ttcccactag caatggctac ccgcaaggtc gcacctgcga tcgctgcagg ttgtgtcatg     540

gtgctcaagc cagctcgact taccccgctg acctcccagt atttgctcag gaccatgctt      600

gatgccggtc ttccagcagg tgtcctcaat gtggtctccg gtgcttccgc ctctgcgatt     660

tccaacccga ttatggaaga cgatcgcctt cgtaaagtct ccttcaccgg ctccacccca 720

gttggccagc agctgctcaa aaaggctgcc gataaagttc tgcgcacctc catggaactt 780

ggtggcaacg cacctttcat tgtcttcgag gacgccgacc tagatctcgc gatcgaaggt 840

gccatgggtg ccaaaatgcg caacatcggc gaagcttgca ccgcagccaa ccgtttctta 900

gtccacgaat ccgtcgccga tgaattcggc cgtcgcttcg ctgcccgcct tgaagagcaa 960

gtcctaggca acggcctcga cgaaggcgtc accgtgggcc ccctggttga ggaaaaagca 1020

cgagacagcg ttgcatcgct tgtcgacgcc gccgtcgccg aaggtgccac cgtcctcacc 1080

ggcggcaagg ccggcacagg tgcaggctac ttctacgaac caacggtgct cacgggagtt 1140

tcaacagatg cggctatcct gaacgaagag atcttcggtc ccgtcgcacc gatcgtcacc 1200

ttccaaaccg aggaagaagc cctgcgtcta gccaactcca ccgaatacgg actggcctcc 1260

tatgtgttca cccaggacac ctcacgtatt ttccgcgtct ccgatggtct cgagttcggc 1320

ctagtgggcg tcaattccgg tgtcatctct aacgctgctg caccttttgg tggcgtaaaa 1380

caatccggaa tgggccgcga aggtggtctc gaaggaatcg aggagtacac ctccgtgcag 1440

tacatcggta tccgggatcc ttacgccggc tag 1473

<210> 66
<211> 1566
<212> DNA
<213> E.coli gabD3

<400> 66
atgatcaaac gtcttccttt aggtccgctg cctaaagaac ttcatcagac tctgcttgat 60

ctgaccgcaa atgcccaaga tgcggcgaaa gtggaggtta tagcgccatt tactggcgag 120

accctcggat ttgtttttga tggtgatgag caagacgtcg agcatgcttt tgcactttca 180

agggcagccc agaaaaagtg ggtgcacacc acggcagtgg aacggaagaa gatcttcctg 240

aagtttcatg atctggtatt gaaaaaccgt gagctgctca tggacatcgt gcagttggaa 300

acaggcaaaa atcgagcatc ggctgccgat gaggtgttgg acgttgcgat caccacccgc 360

ttctacgcaa acaatgcagg aaagttttta aatgacaaga aacgccccgg cgcgcttccg 420

atcatcacga aaaacacaca acagtatgtg cccaagggag tggtcggcca gatcacgccg 480

tggaattacc ctttaacttt gggagtatct gatgctgttc cggcgctgct ggcaggaaac 540

gcagtggtgg ctaaacctga cctcgcgaca cctttctcct gcttgatcat ggtgcacctg 600

ctcattgaag ccggtctgcc gcgtgatttg atgcaggttg tcaccggccc tggcgatatt 660

gttggcggtg cgattgcagc tcagtgtgat ttcctcatgt tcactggatc cacggccacg 720

ggccggatct tgggtcggac aatgggtgag cgtttggtgg gtttctctgc ggaattaggc 780

ggaaagaacc ctcttattgt ggccaaggat gcagatctgg acaaggtgga agctgagctt 840

ccgcaggcgt gtttttccaa ctcggggcaa ttgtgtgtct ccactgaacg tatttatgtc 900

gaggaagacg tgtacgagga ggtgattgca cggtttagca aggcggcgaa agccatgtcc 960

attggtgccg gatttgagtg gaaatatgag atgggttcgt tgatcaatca ggcgcagctg 1020

gatcgggtga gcacctttgt tgatcaggct aaagctgcgg gcgccacggt gctgtgcggt 1080

ggcaagtcac gccctgatat tggtcccttc ttctatgagc ccacggtatt ggcggatgtc 1140

ccagagggca ccccactgct cacggaggaa gtcttcggcc cggtggtgtt catcgaaaag 1200

gtagccacac tggaagaagc cgtcgataag gcaaatggca cgccctacgg cctgaatgcg 1260

tccgtctttg ggtcgtcgga aaccggcaat cttgttgcag gccagctgga agctggcggt 1320

atcggtatta atgatggcta cgccgcgacg tgggcgagcg tgtccacgcc tctgggtggc 1380

atgaagcagt cggggctggg gcaccgccat ggtgcggagg gaattacaaa atatgcggag 1440

atccgaaaca tcgcggagca gcgctggatg tctatgcgtg ggccggccaa aatgccgcga 1500

aaggtgtact cagacaccgt ggccacagcg ctaaagctgg gcaaaatctt taaagttttg 1560

ccgtag 1566

<210> 67
<211> 426
<212> PRT
<213> E.coli gabT

<400> 67
Met Asn Ser Asn Lys Glu Leu Met Gln Arg Arg Ser Gln Ala Ile Pro
1               5                   10                  15

Arg Gly Val Gly Gln Ile His Pro Ile Phe Ala Asp Arg Ala Glu Asn
            20                  25                  30

Cys Arg Val Trp Asp Val Glu Gly Arg Glu Tyr Leu Asp Phe Ala Gly
        35                  40                  45

Gly Ile Ala Val Leu Asn Thr Gly His Leu His Pro Lys Val Val Ala
    50                  55                  60

Ala Val Glu Ala Gln Leu Lys Lys Leu Ser His Thr Cys Phe Gln Val
65                  70                  75                  80

Leu Ala Tyr Glu Pro Tyr Leu Glu Leu Cys Glu Ile Met Asn Gln Lys
                85                  90                  95

Val Pro Gly Asp Phe Ala Lys Lys Thr Leu Leu Val Thr Thr Gly Ser
            100                 105                 110

Glu Ala Val Glu Asn Ala Val Lys Ile Ala Arg Ala Ala Thr Lys Arg
        115                 120                 125

Ser Gly Thr Ile Ala Phe Ser Gly Ala Tyr His Gly Arg Thr His Tyr
    130                 135                 140

Thr Leu Ala Leu Thr Gly Lys Val Asn Pro Tyr Ser Ala Gly Met Gly
145                 150                 155                 160

Leu Met Pro Gly His Val Tyr Arg Ala Leu Tyr Pro Cys Pro Leu His
                165                 170                 175

Gly Ile Ser Glu Asp Asp Ala Ile Ala Ser Ile His Arg Ile Phe Lys
            180                 185                 190

Asn Asp Ala Ala Pro Glu Asp Ile Ala Ala Ile Val Ile Glu Pro Val
        195                 200                 205

Gln Gly Glu Gly Gly Phe Tyr Ala Ser Ser Pro Ala Phe Met Gln Arg
    210                 215                 220

Leu Arg Ala Leu Cys Asp Glu His Gly Ile Met Leu Ile Ala Asp Glu
225                 230                 235                 240

Val Gln Ser Gly Ala Gly Arg Thr Gly Thr Leu Phe Ala Met Glu Gln
                245                 250                 255

Met Gly Val Ala Pro Asp Leu Thr Thr Phe Ala Lys Ser Ile Ala Gly
            260                 265                 270

Gly Phe Pro Leu Ala Gly Val Thr Gly Arg Ala Glu Val Met Asp Ala
        275                 280                 285

Val Ala Pro Gly Gly Leu Gly Gly Thr Tyr Ala Gly Asn Pro Ile Ala
    290                 295                 300

Cys Val Ala Ala Leu Glu Val Leu Lys Val Phe Glu Gln Glu Asn Leu
305                 310                 315                 320

Leu Gln Lys Ala Asn Asp Leu Gly Gln Lys Leu Lys Asp Gly Leu Leu
                325                 330                 335

Ala Ile Ala Glu Lys His Pro Glu Ile Gly Asp Val Arg Gly Leu Gly
            340                 345                 350

Ala Met Ile Ala Ile Glu Leu Phe Glu Asp Gly Asp His Asn Lys Pro
        355                 360                 365

Asp Ala Lys Leu Thr Ala Glu Ile Val Ala Arg Ala Arg Asp Lys Gly
    370                 375                 380

Leu Ile Leu Leu Ser Cys Gly Pro Tyr Tyr Asn Val Leu Arg Ile Leu
385                 390                 395                 400

Val Pro Leu Thr Ile Glu Asp Ala Gln Ile Arg Gln Gly Leu Glu Ile
                405                 410                 415

Ile Ser Gln Cys Phe Asp Glu Ala Lys Gln
            420                 425


<210>    68
<211>    466
<212>    PRT
<213>    E.coli Glutamate decarboxylase alpha

<400>    68
Met Asp Gln Lys Leu Leu Thr Asp Phe Arg Ser Glu Leu Leu Asp Ser
1               5                   10                  15

Arg Phe Gly Ala Lys Ala Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

His Thr
465

<210>    69
<211>    466
<212>    PRT
<213>    E.coli Glutamate decarboxylase beta (gadB)

<400>    69
Met Asp Lys Lys Gln Val Thr Asp Leu Arg Ser Glu Leu Leu Asp Ser
 1               5                  10                  15

Arg Phe Gly Ala Lys Ser Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys

            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

His Thr
465

## Description

### BACKGROUND

### 1. Field

The present disclosure relates to a genetically modified bacterial cell having enhanced succinic acid production and a method for producing succinic acid by using the same.

### 2. Description of the Related Art

*Corynebacterium* is a gram positive bacteria used for the production of amino acids such as glutamate, lysine, and threonine. *Corynebacterium* has advantages as an industrial strain because it can grow in a variety of conditions and environments, its genome structure is stable, and it poses little risk as an environmental hazard. *Corynebacterium glutamicum* is an aerobic bacteria. In anaerobic conditions, the metabolic processes of *Corynebacterium glutamicum,* other than those necessary to produce minimum energy for survival, cease. As a result, lactic acid, acetic acid, and succinic acid. are produced and released from *Corynebacterium glutamicum.*

Tricarboxylic acid (TCA) cycle is a metabolic process utilized by organisms such as *Corynebacterium* to produce energy and intermediate products. The intermediates produced in the TCA cycle are converted into useful chemical materials via various metabolic processes in cells. Succinic acid is a kind of dicarboxylic acid used as a raw material for the manufacture of biodegradable polymers, medicines, foods and cosmetics. Most industrially used succinic acid is synthesized from n-butane and acetylene derived from crude oil or liquefied natural gas. Only a small amount of succinic acid used for special purposes such as medicines and foods has been produced by fermentation processes using a microorganism. A chemical synthesis process generally generates a large amount of hazardous materials, and uses fossil fuels, which are rapidly depleted. Thus, it is exists a need to develop a method for efficiently producing succinic acid using microorganisms.

### SUMMARY

Provided is a genetically engineered bacterial cell wherein the activity of a pathway in the cell of converting α-ketoglutarate (AKG) into succinate semialdehyde (SSA) and/or the activity of succinyl semialdehyde dehydrogenase (SSADH) in the cell is increased as compared to the activity in a parent cell. The pathway of converting α-ketoglutarate (AKG) into SSA includes α-ketoglutarate decarboxylase (KGDC); glutamate:succinate semialdehyde transaminase and glutamate decarboxylase; or a combination thereof, wherein the KGDC catalyzes the conversion of α-ketoglutarate into SSA, and the SSADH catalyzes the conversion of SSA into succinic acid. Thus, in one aspect, the bacterial cell is engineered to increase the activity of succinyl semialdehyde dehydrogenase (SSADH), α-ketoglutarate decarboxylase (KGDC), glutamate:succinate semialdehyde transaminase, glutamate decarboxylase, or a combination thereof, in the genetically engineered bacterial cell as compared to a parent cell. According to another aspect of the present invention, provided is a genetically engineered bacterial cell in which the copy number of a gene encoding at least one selected from the group consisting of a pathway of converting α-ketoglutarate into SSA; and succinyl semialdehyde dehydrogenase is increased, or the expression of the gene(s) is increased, as compared to a parent cell. In a related aspect, the engineered bacterial cell comprises an increased copy number of a gene encoding succinyl semialdehyde dehydrogenase (SSADH), encoding α-ketoglutarate decarboxylase (KGDC), encoding glutamate:succinate semialdehyde transaminase, encoding glutamate decarboxylase, or encoding a combination thereof, as compared to a parent cell.

Further provided is a method for producing succinic acid by using the genetically engineered bacterial cell. The method comprises culturing the genetically engineered bacterial cell, whereby the cell produces succinic acid; and recovering succinic acid from the culture.

Also provided is a method of improving succinic acid production by a bacterial cell, the method comprising increasing the activity of succinyl semialdehyde dehydrogenase (SSADH), α-ketoglutarate decarboxylase (KGDC), glutamate:succinate semialdehyde transaminase, glutamate decarboxylase, or a combination thereof, in the cell. In one aspect, the activity of one or more of the enzymes is increased by increasing the copy number of one or more genes encoding the one or more enzymes.

Additional aspects will be set forth in part in the description which follows and, in part, will be apparent from the description, or may be learned by practice of the presented embodiments.

### BRIEF DESCRIPTION OF THE DRAWINGS

These and/or other aspects will become apparent and more readily appreciated from the following description of the embodiments, taken in conjunction with the accompanying drawings of which:
FIG. 1 is an expression vector map of a pGSK+ vector;
FIG. 2 is an expression vector a map of pGS-Term vector;
FIG. 3 is an expression vector map of pGS-EX4 vector; and
FIG. 4 is an expression vector map of a vector used for the introduction of a gene encoding α-ketoglutarate decarboxylase and a gene encoding succinyl semialdehyde dehydrogenase into a microorganism.

### DETAILED DESCRIPTION

An aspect of the present invention provides a genetically engineered bacterial cell, wherein the activity of a pathway in the cell of converting α-ketoglutarate (AKG) into succinate semialdehyde (SSA) and/or the activity of succinyl semialdehyde dehydrogenase (SSADH) in the cell is increased as compared to the activity in a parent cell. Another embodiment provides a genetically engineered bacterial cell, wherein the activity of α-ketoglutarate decarboxylase (KGDC) which catalyzes the conversion of α-ketoglutarate into SSA; of glutamate:succinate semialdehyde transaminase; of glutamate decarboxylase; or of a combination thereof is increased as compared to a parent cell. The glutamate:succinate semialdehyde transaminase may catalyze the following chemical reaction:

4-aminobutanoate + 2-oxoglutarate → succinate semialdehdye + L-glutamate.

The glutamate:succinate semialdehyde transaminase may be an enzyme included in EC 2.6.1.19. The glutamate:succinate semialdehyde transaminase may be an *Escherichia coli*-derived gabT (SEQ ID NO: 67) or an enzyme derived from S. *cerevisiae.* The glutamate decarboxylase may convert glutamate into CO₂ and 4-aminobutyrate. The glutamate decarboxylase may be included in EC 4.1.1.15. The glutamate decarboxylase may be an *Escherichia coli*-derived glutamate decarboxylase alpha (SEQ ID NO: 68) or an *Escherichia coli*-derived glutamate decarboxylase beta (SEQ ID NO: 69), or an enzyme derived from S. *cerevisiae.*

As used herein the term "increase in activity" or "increased activity" of a cell, pathway, enzyme, or polypeptide may refer to a detectable increase in the activity thereof. "Increase in activity" or "increased activity" may also refer to an activity level of a modified (e.g., genetically engineered) cell, pathway, protein, or enzyme that is higher than that of a comparable cell, pathway, protein, or enzyme of the same type, such as a cell, pathway, protein, or enzyme that does not have a given modification (e.g., the original or parent or "wild-type" cell, pathway, protein, or enzyme). The comparable cell may be an unmodified parent cell lacking a specific genetic modification that produced the genetically engineered cell. For example the activity of the cell, pathway, enzyme, or polypeptide may have activity level that is increased by about 5%, about 10%, about 15%, about 20%, about 30%, about 50%, about 60%, about 70%, or about 100% in comparison with the same activity of the unmodified cell, pathway, enzyme, or polypeptide. An activity of a polypeptide may be measured by using a method known to those of ordinary skill in the art. "Activity increase" or "increased activity" of a pathway may be caused by an increase of the activity of an enzyme or a polypeptide related to the pathway.

The activity increase of the enzyme or peptide may be achieved by an increased expression of the gene encoding the enzyme or peptide. The expression increase may be caused by an introduction of a polynucleotide encoding the polypeptide, by an increase of the number of copies of the encoding gene, or by mutation of a regulatory region of the polynucleotide. A polynucleotide which is introduced externally or whose copy number is increased may be endogenous or exogenous to the cell. The endogenous gene refers to a gene which has existed on a genetic material included in a microorganism. The exogenous gene refers to a gene which is introduced to a host cell by a method such as integration to a host cell genome. An introduced gene may be homologous or heterologous to the host cell.

The term used herein "increase of activity" or "increased activity" of a pathway, an enzyme, or a polypeptide also includes causing a cell to have an activity which is not native or preexisting in the cell by genetic engineering.

The term "copy number increase," as used herein, may refer to an increase of copy number by the introduction of a gene or amplification of a gene, and may be achieved by genetically engineering a cell to have a gene that does not naturally exist in the cell. The introduction of a gene may be mediated by a vehicle such as a vector. The introduction may be a transient introduction in which the gene is not integrated to a genome, or the gene may be inserted into the cellular genome. The introduction may be performed, for example, by introducing a vector into the cell, wherein the vector comprises a polynucleotide encoding a target polypeptide, and expressing the vector in the cell separately from the genome, or by integrating the polynucleotide into the genome.

The term "gene" refers to a nucleic acid fragment expressing a specific protein and may include a coding region as well as regulatory sequences such as a 5'-non coding sequence or a 3'-non coding sequence.

The term "heterologous" means "foreign," or "not native." The term "homologous" means "native."

The term "secretion" means transport of a material from the inside of a cell to a periplasmic space or an extracellular environment.

The terms "cell," "strain," or "microorganism" may be used interchangeably and include bacteria.

The term "activity decrease" or "decreased activity" of an cell, pathway, enzyme or polypeptide (e.g., a modified or genetically engineered cell, pathway, enzyme, or polypeptide) refers to an activity level that is lower than the activity level measured in a comparable cell, pathway, enzyme, or polypeptide (e.g., a cell, pathway, enzyme, or polypeptide of the same kind that does not contain the modification or genetic engineering), or that the activity is absent. For example, the activity of a modified or genetically engineered cell, pathway, enzyme, or polypeptide may be decreased by about 20%, about 30%, about 40%, about 50%, about 55%, about 60%, about 70%, about 75%, about 80%, about 85%, about 90%, about 95%, or about 100% in comparison with to an unmodified cell, pathway, enzyme, or polypeptide of the same type. Decreased enzyme activity may be verified by using a method known to those of ordinary skill in the art for measuring an enzyme activity. The decrease in activity may be caused by the expression of an enzyme that is mutated so as to exhibit no activity or a decreased activity compared to the activity level of an unmodified enzyme (e.g., enzyme produced by an unmodified cell), or by the under-expression (or lack of expression) of a gene encoding an enzyme as compared to the expression of an unmodified gene of the same type (e.g., expression of the same gene in an unmodified cell).

The decrease of enzyme activity may be caused by deletion or disruption of a gene encoding the enzyme. The term "deletion" or "disruption" refers to mutating, substituting, or deleting a part of a gene or an entire gene, or a part of a regulatory element or an entire regulatory element such as a prompter region and a terminator region of the gene or inserting at least one base to the gene so that the gene may not be expressed, or the amount of expression may be decreased, or the enzyme activity may not be shown or decreased even when the gene is expressed. The deletion or disruption of a gene may be achieved through genetic engineering such as homologous recombination, mutation, or molecular evolution. When a cell includes multiple copies of a same gene or two or more paralogs of different polypeptides, one or more genes may be deleted or disrupted.

The term "sequence identity" of a nucleic acid or a polypeptide used herein refers to a degree of similarity of base groups or amino acid residues between two aligned sequences, when the two sequences are aligned to match each other to the greatest extent possible, at corresponding positions. The sequence identity is a value that is measured by aligning to an optimum state and comparing the two sequences at a particular region of interest, wherein a part of the sequence within the particular modified cell may be added or deleted compared to a reference sequence. A sequence identity percentage may be calculated, for example, by 1) comparing the two sequences aligned within the whole comparing region to an optimum 2) obtaining the number of matched locations by determining the number of locations represented by the same amino acids of nucleic acids in both of the sequences, 3) dividing the number of the matched locations by the total number of the locations within the comparing region (i.e., a range size), and 4) obtaining a percentage of the sequence identity by multiplying 100 to the result. The sequence identity percent may be determined by using a common sequence comparing program, for example, BLASTN (NCBI), CLC Main Workbench (CLC bio), MegAlign™ (DNASTAR Inc).

In confirming that different polypeptides or polynucleotides have the same or similar function or activity, sequence identities may be used. For example, the sequence identities may include about 50% or greater, about 55% or greater, about 60% or greater, about 65% or greater, about 70% or greater, about 75% or greater, about 80% or greater, about 85% or greater, about 90% or greater, about 95% or greater, about 96% or greater, about 97% or greater, about 98% or greater, about 99% or greater, or 100%.

The cell may have a succinic acid-producing ability. The cells may have an increase in succinic acid-producing ability as compared to a parent cell (e.g., non-genetically engineered cells), by being genetically engineered such that the activity of a pathway of converting α-ketoglutarate into SSA; and/or succinyl semialdehyde dehydrogenase in the cell is increased compared to a parent cell (e.g., non-genetically engineered cell).

The cells may be capable of producing succinic acid in aerobic, anaerobic or microaerobic conditions. The microaerobic condition may refer to a condition where oxygen of a level lower than the atmospheric oxygen concentration is dissolved into a culture medium. The lower level of oxygen refers to from about 0.1% to about 10%, about 1% to about 9%, about 2% to about 8%, about 3% to about 7%, or about 4% to about 6% of the atmospheric oxygen concentration. The cells may produce succinic acid via oxidative TCA cycle as well as via reductive TCA cycle. The genetically engineered cells may have an increase in succinic acid-producing ability as compared to the non-genetically engineered cells by producing succinic acid via oxidative TCA cycle. The genetically engineered cells may have an increase in succinic acid-producing ability as compared to the non-genetically engineered cells by producing succinic acid for example, in a microaerobic condition via oxidative TCA cycle.

The cell may be *Corynebacterium.* The cells may be, for example, *Corynebacterium glutamicum, Corynebacterium thermoaminogenes, Brevibacterium flavum* or *Brevibacterium lactofermentum.*

The activity may be increased by an increase in the copy number of at least one gene encoding a polypeptide used in the pathway of converting α-ketoglutarate into SSA, and/or an increase in the copy number of a gene encoding succinyl semialdehyde dehydrogenase. An "increase" in copy number of the genetically engineered cell as compared to an unmodified "parent" cell includes, for instance, introduction of a gene into the cell that does not exist in the unmodified "parent" cell. The gene encoding a polypeptide used in the pathway of converting α-ketoglutarate into SSA may include a gene encoding KGDC which catalyzes the conversion of α-ketoglutarate into SSA; a gene encoding glutamate:succinate semialdehyde transaminase and a gene encoding glutamate decarboxylase; or a combination thereof. The increase in copy number may be due to the introduction or amplification of the gene. The introduction may be mediated by a vehicle such as a vector. The introduction may be transient where the gene is not integrated into its chromosome or the gene may be inserted into its chromosome. The introduction may be achieved by introducing a vector where a polynucleotide encoding KGDC or succinyl semialdehyde dehydrogenase is inserted into a cell, and replicating the vector in the cell or integrating the polynucleotide into the chromosome. The gene may include a regulatory sequence in addition to a nucleotide sequence encoding a protein. The gene may be endogenous or exogenous.

In addition, the activity may be increased by modification of regulatory sequences for expressing the gene(s). The regulatory sequence may be a promoter sequence for expressing the gene or a transcription terminator sequence. The promoter may be P₁₈₀ promoter, or P₄₅ promoter. The transcription terminator may be rrnBT terminator from a E.coli. The modification may a replacement of the endogenous promoter or terminator with the exogenous promoter or terminator. The regulatory sequence may also be a nucleotide sequence encoding a motif which can influence the expression of the gene. The motif may be, for example, a secondary structure-stabilizing motif, an RNA destabilizing motif, a splice-activating motif, a polyadenylation motif, an adenine-rich sequence, or an endonuclease recognition site.

In addition, the activity may be increased by mutation of a polypeptide and/or of a gene encoding a polypeptide that changes the amino acid sequence of at least one polypeptide used in a pathway of converting α-ketoglutarate into SSA; and/or succinyl semialdehyde dehydrogenase. The modification may be produced by deletion, insertion, substitution of a nucleotide sequence in a protein encoding region, or combinations thereof. The modification may, for example, increase the affinity of an enzyme included in the pathway of converting α-ketoglutarate into SSA or succinyl semialdehyde dehydrogenase for its substrate. Accordingly, the modification may increase the specific activity of the enzyme itself.

The KGDC may be an enzyme classified to EC.4.1.1.71. For example, the KGDC is derived from *Corynebacterium* sp., *Rhodococcus* sp., *Mycobacterium* sp., or *Escherichia* sp. The gene encoding the KGDC may be derived from, for example, *Corynebacterium glutamicum, Corynebacterium callunae, Corynebacterium efficiens, Corynebacterium ulcerans, Corynebacterium halotolerans, Corynebacterium pseudotuberculosis, Corynebacterium durum, Corynebacterium striatum, Rhodococcus pyridinivorans, Rhodococcus ruber, Mycobacterium abscessus, Mycobacterium smegmatis, Escherichia coli, Escherichia fergusonii,* or combinations thereof. The gene encoding the KGDC may be, for example, a polynucleotide encoding an amino acid sequence having a sequence identity of 95% or higher with the amino acid sequence of SEQ ID NO:1 or SEQ ID NO: 2. The gene encoding the KGDC may have, for example, a nucleotide sequence having a sequence identity of 95% or higher with the nucleotide sequence of SEQ ID NO: 3 or SEQ ID NO: 4.

The succinyl semialdehyde dehydrogenase (SSADH) may be an enzyme classified to EC.1.2.1.24, EC.1.2.1.79, or EC.1.2.1.16. The SSADH may be dependent on NAD+-, NADP+, or both. The SSADH may be CoA-independent. For example, the SSADH may be derived from *Corynebacterium* sp., *Rhodococcus* sp., *Gordonia* sp., *Mycobacterium* sp., or *Escherichia* sp.. The SSADH may be an *Escherichia coli*-derived gabD1, gabD2, or gabD3. For example, the gene encoding the SSADH may be a polynucleotide encoding an amino acid sequence having a sequence identity of 95% or higher with an amino acid sequence of SEQ ID NOS: 5 (gabD2), 63 (gabD1), and 64 (gabD3). The gene encoding SSADH may have a nucleotide sequence having a sequence identity of 95% or higher with a polynucleotide of SEQ ID NOS: 6 (gabD2), 65 (gabD1), and 66 (gabD3).

The cell may be characterized in that the gene encoding succinyl-CoA synthetase (SucCD), which catalyzes the conversion of succinic acid into succinyl-CoA, is inactivated or attenuated. The succinyl-CoA synthetase may be classified as EC.6.2.1.5. The gene encoding succinyl-CoA synthetase may be, for example, a polynucleotide encoding an amino acid sequence having a sequence identity of 95% or higher with an amino acid sequence of SEQ ID NO:7 or SEQ ID NO: 8.

The cell may be characterized in that the gene encoding L-lactate dehydrogenase (LDH) which catalyzes the conversion of pyruvate into lactate, the gene encoding pyruvate oxidase (PoxB) which catalyzes the conversion of pyruvate into acetate, the gene encoding phosphotransacetylase (PTA) which catalyzes the conversion of acetyl-CoA into acetyl phosphate, the gene encoding acetate kinase (AckA) which catalyzes the conversion of acetyl phosphate into acetate, the gene encoding acetate CoA transferase (ActA) which catalyzes the conversion of acetate into acetyl-CoA, or a combination thereof is inactivated or attenuated. The LDH may be an enzyme classified as EC.1.1.1.27. The LDH may have, for example, an amino acid sequence having a sequence identity of 95% or higher with an amino acid sequence of SEQ ID NO:9. The PoxB may be an enzyme classified as EC.1.2.5.1. The PoxB may have, for example, an amino acid sequence having a sequence identity of 95% or higher with an amino acid sequence of SEQ ID NO: 10. The PTA may be an enzyme classified as EC.2.3.1.8. The PTA may have, for example, an amino acid sequence having a sequence identity of 95% or higher with an amino acid sequence of SEQ ID NO: 11. The AckA may be an enzyme classified as EC.2.7.2.1. The AckA may have, for example, an amino acid sequence having a sequence identity of 95% or higher with an amino acid sequence of SEQ ID NO: 12. The ActA may be an enzyme classified as EC.2.8.3.9. The ActA may have, for example, an amino acid sequence having a sequence identity of 95% or higher with an amino acid sequence of SEQ ID NO:13. When these genes or combinations thereof are inactivated or attenuated, the activity of converting pyruvate into lactate, the activity of converting pyruvate into acetate, or combinations thereof in the cell may be lost or decreased, which in turn increases the metabolic introduction of pyruvate into the TCA cycle and hence it conversion into succinic acid.

The term, "inactivation" or "inactivated" used herein, may refer to a gene (or act of generating a gene) which cannot be expressed or encodes a protein having no activity when expressed. The term, "attenuation" or "attenuated" used herein, may refer to a gene (or act of generating a gene) whose expression level is reduced as compared to the expression level of the same gene in a non-engineered bacterial strain or a parental bacterial strain of the same type (e.g., a "wild-type" strain), or a gene which encodes a protein with decreased activity as compared to the activity of the same protein in a non-engineered bacterial strain or a parental bacterial strain of the same type (e.g., a "wild-type" strain). The inactivation or attenuation may be caused by a genetic modification, for example, substitution or addition mutation, or by deletion or all or part of the gene. Any mode of modification may be used, such as homologous recombination. The inactivation or attenuation may be achieved, for example, via transformation by inserting a vector including a partial nucleotide sequence of a gene by inserting into the cell, culturing the cell to induce a homologous recombination between the partial nucleotide sequence and the sequence of an endogenous gene, and selecting a cell where the homologous recombination occurred by a selective marker.

The cell may be one with increased activity of pyruvate carboxylase (PYC) which catalyzes the conversion of pyruvate into oxaloacetate. The increased activity may be achieved, for example, by the introduction of a gene encoding pyruvate carboxylase, which has an increased specific activity due to a mutation. The mutation may include substitution, addition, deletion, or combinations thereof. The substitution may be, for example, a substitution of the 458^{th} amino acid of SEQ ID NO: 14 from proline to serine.

According to another aspect of the present invention, provided is a genetically engineered bacterial cell, wherein the copy number of at least one selected from the group consisting of a gene encoding a polypeptide used in a pathway of converting α-ketoglutarate into SSA; and/or a gene encoding SSADH is increased, or the expression of the gene(s) is increased as compared to a non-genetically engineered cell, wherein the pathway of converting α-ketoglutarate into SSA includes KGDC which catalyzes the conversion of α-ketoglutarate into SSA; glutamate:succinate semialdehyde transaminase and glutamate decarboxylase; or a combination thereof, and wherein the SSADH catalyzes the conversion of succinyl semialdehyde into succinic acid. The gene encoding a polypeptide used in the pathway of converting α-ketoglutarate into SSA may be a gene encoding KGDC which catalyzes the conversion of α-ketoglutarate into SSA; a gene encoding glutamate: succinate semialdehyde transaminase and a gene encoding glutamate decarboxylase; or a combination thereof.

The cell may be *Corynebacterium.* The cell may be, for example, *Corynebacterium glutamicum, Corynebacterium thermoaminogenes, Brevibacterium flavum* or *Brevibacterium lactofermentum.*

The copy number may be increased by introduction or amplification of the gene(s). The details of the introduction of the gene are the same as described above. The gene introduced may be endogenous or exogenous. The gene encoding the KGDC may be one encoding an enzyme classified as EC.4.1.1.71.

The gene encoding the KGDC may be derived from *Corynebacterium* sp. or *Escherichia* sp. including *Escherichia coli.* For example, the gene may be one encoding an amino acid sequence having a sequence identity of 95% or higher with an amino acid sequence of SEQ ID NO:1 or SEQ ID NO:2. The gene encoding the SSADH may be one encoding an enzyme classified as EC.1.2.1.16. The gene encoding the SSADH may be, for example, one derived from *Corynebacterium* sp., *Rhodococcus* sp., *Gordonia* sp., *Mycobacterium* sp., *Anthrobacter* sp., or *Escherichia* sp.. For example, the gene may be one encoding an amino acid sequence having a sequence identity of 95% or higher with an amino acid sequence of SEQ ID NOS:5 (gabD2), 63 (gabD1), and 64 (gabD3). The gene encoding the SSADH may have a nucleotide sequence having a sequence identity of 95% or higher with a polynucleotide of SEQ ID NOS: 6(gabD2), 65 (gabD1), and 66 (gabD3).

The expression of the gene(s) may be increased by modification of regulatory sequences for expressing the gene(s). The details of the regulatory sequence are the same as described above.

The cell may be one where a gene encoding succinyl-CoA synthetase is inactivated or attenuated. The cell may be one where L-lactate dehydrogenase gene, pyruvate oxidase gene, phosphotransacetylase gene, acetate kinase gene, acetate CoA transferase gene, or combinations thereof is inactivated or attenuated. The cell may be one with increased activity of pyruvate carboxylase which catalyzes the conversion of pyruvate into oxaloacetate. The details of such modifications are as described herein with respect to other embodiments.

According to further aspect of the present invention, provided is a method for producing succinic acid comprising culturing cell genetically modified cell as described above in a cell culture medium, whereby the cell produces succinic acid, and recovering succinic acid from the culture is provided. Other aspects of the genetically modified cell useful in this method are as described above with respect to other embodiments.

The culturing may be performed according to a suitable culture medium and conditions known in the art. A skilled person in the art can easily adjust the culture medium and conditions according to the microorganism to be selected. The culture method may include a batch culture, a continuous culture and a fed-batch culture.

The culture medium can include various carbon sources, nitrogen sources and a trace amount of components. The carbon sources may include, for example, a carbohydrate such as glucose, sucrose, lactose, fructose, maltose, starch, and cellulose; a fatty acid such as soybean oil, sunflowers oil, castor oil, coconut oil, palmitic acid, stearic acid, and linoleic acid; an alcohol such as glycerol and ethanol; an organic acid such as acetic acid; and/or combinations thereof. The culturing may be performed, for example, by using glucose as a carbon source. The nitrogen source may include, for example, an organic nitrogen source such as peptone, yeast extract, meat extract, malt extract, corn steep liquor (CSL), and soybean wheat; an inorganic nitrogen source such as urea, ammonium sulfate, ammonium chloride, ammonium phosphate, ammonium carbonate, and ammonium nitrate; and/or combinations thereof. The culture medium may include as a phosphorous source, for example, potassium dihydrogen phosphate, dipotassium hydrogen phosphate and its corresponding sodium-containing salt, a metal salt such as magnesium sulfate or iron sulfate. In addition, the culture medium may include amino acids, vitamins, and suitable precursors. The culture medium or individual components may be added to a culture broth in the form of a batch culture or a continuous culture.

Additionally, compounds such as ammonium hydroxide, potassium hydroxide, ammonia, phosphoric acid, and sulfuric acid may be added in an appropriate manner into a culturing broth during culture to adjust the pH of the culturing broth. Furthermore, an anti-foaming agent such as fatty acid polyglycol ester may be used to prevent generation of foams during culture.

The cell can be cultured in aerobic, microaerobic, or anaerobic conditions. The microaerobic condition refers to a culturing condition where a lower level of oxygen than that in the atmosphere is dissolved in a culture medium. The lower level of oxygen is, for example, from about 0.1 % to about 10%, about 1% to about 9%, about 2% to about 8%, about 3% to about 7%, or about 4 to about 6%. Additionally, the microaerobic condition may be, for example, a culturing condition where the concentration of oxygen dissolved in a culture medium is from about 0.9 ppm to about 3.6 ppm, for example, from about 0.9 ppm to about 2.6 ppm, from about 0.9 ppm to about 1.6 ppm, from 1.0 ppm to about 3.6 ppm, from about 2.0 ppm to about 3.6 ppm, or from about 3.0 ppm to about 3.6 ppm. The culturing temperature may be, for example, from about 20°C to about 45°C, or from about 25°C to about 40°C. The cultivation may be continued until a desirable amount of target succinic acid is obtained.

The succinic acid can be recovered by using a conventional separation and purification method. The recovery can be also made by centrifugation, ion exchange chromatography, filtration, precipitation, or combinations thereof. For example, the culture can be centrifuged to remove biomass, and the resulting supernatant can be separated by ion exchange chromatography.

Provided is a method of improving succinic acid production by a bacterial cell, the method comprising increasing the activity of succinyl semialdehyde dehydrogenase (SSADH), α-ketoglutarate decarboxylase (KGDC), glutamate:succinate semialdehyde transaminase, glutamate decarboxylase, or a combination thereof, in the cell. Suitable means for increasing the activity of succinyl semialdehyde dehydrogenase (SSADH), α-ketoglutarate decarboxylase (KGDC), glutamate:succinate semialdehyde transaminase, glutamate decarboxylase, or a combination thereof are as described, above.

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description.

### Example 1: Preparation of a bacterial strain deprived of synthetic pathway for lactate and acetate

### (1) Construction of a replacement vector

The L-lactate dehydrogenase (ldh), pyruvate oxidase (poxB), phosphotransacetylase (pta), acetate kinase (ackA), and acetate CoA transferase (actA) genes of Corynebacterium glutamicum (C. glutamicum; CGL) ATCC 13032 were inactivated by homologous recombination. The genes were inactivated by using pK19 mobsacB (ATCC 87098) vector, and the two homologous domains used for recombination were obtained via PCR amplification using the genomic DNA of CGL ATCC 13032 as a template.

The two homologous domains for removing ldh gene were obtained via PCR amplification by using a primer set of ldhA_5'_HindIII (SEQ ID NO: 15) and ldhA_up_3'_XhoI (SEQ ID NO: 16) as an upstream domain of the gene, and a primer set of ldhA_dn_5_Xhol (SEQ ID NO: 17) and ldhA_3'_EcoRI (SEQ ID NO: 18) as a downstream of the gene, respectively. The PCR amplification was performed by repeating a cycle 30 times, wherein the cycle consisted of denaturation at 95°C for 30 seconds, annealing at 55°C for 30 seconds, and extension at 72°C for 30 seconds. Hereinafter, all PCR amplifications were performed in the same manner. The obtained PCR amplification product was cloned in between Hindlll and EcoRI of pK19 mobsacB, and a pK19_Δldh vector was constructed therefrom.

The two homologous domains for removing poxB gene were obtained via PCR amplification by using a primer set of poxB 5' H3 (SEQ ID NO: 19) and DpoxB_up 3' (SEQ ID NO:20) as an upstream domain of the gene, and a primer set of DpoxB_dn 5' (SEQ ID NO:21) and poxB 3' E1 (SEQ ID NO: 22) as a downstream domain of the gene, respectively. The obtained PCR amplification product was cloned in between HindIII and EcoRI of pK19 mobsacB, and a pK19_ΔpoxB vector was constructed therefrom.

The two homologous domains for removing pta-ackA gene were obtained via PCR amplification by using a primer set of pta 5' H3 (SEQ ID NO: 23) and Dpta_up_R1 3' (SEQ ID NO: 24) as an upstream domain of the gene, and a primer set of DackA_dn_R1 5' (SEQ ID NO: 25) and ackA 3' Xb (SEQ ID NO: 26) as a downstream domain of the gene, respectively. The obtained PCR amplification product was cloned in between HindIII and Xbal of pK19 mobsacB, and a pK19_Δpta_ackA vector was constructed therefrom.

The two homologous domains for removing actA gene were obtained via PCR amplification by using a primer set of actA 5' Xb (SEQ ID NO: 27) and DactA_up_R4 3'(SEQ ID NO: 28) as an upstream domain of the gene, and a primer set of DactA_dn_R4 5' (SEQ ID NO: 29) and actA 3' H3 (SEQ ID NO: 30) as a downstream domain of the gene, respectively. The obtained PCR amplification product was cloned in between Xbal and Hindlll of pK19 mobsacB, and a pK19_ΔactA vector was constructed therefrom.

### (2) Preparation of CGL (Δldh, ΔpoxB, Δpta-ackA, ΔactA)

The replacement vectors were introduced together into C. glutamicum ATCC13032 via electroporation. The transformed cells were plated out on an LBHIS agar plate containing 25 µg/mL of kanamycin and incubated at 30°C. The LBHIS agar plate contained 25 g/L of Difco LBTM broth, 18.5 g/L of brain-heart infusion broth, 91 g/L of D-sorbitol and 15 g/L of agar. Hereinafter, all the compositions of LBHIS media are prepared in the same manner. The colonies formed thereon were cultured at 30°C in BHIS medium (pH 7.0) containing 37 g/L of brain heart infusion powder and 91 g/L of D-sorbitol, and the culture broth was plated out on an LB/Suc10 agar plate, incubated at 30°C, and those with double crossover were selected. The LB/Suc10 agar plate contained 25 g/L of Difco LBTM broth, 15 g/L of agar, and 100 g/L of sucrose.

Genomic DNA was separated from the selected colonies, and the presence of any deletions in the genes was examined. In order to confirm the presence of any deletions in ldh gene, a primer set of ldhA_5'_HindIII and ldhA_3'_EcoRI was used. In order to confirm the presence of any deletions in poxB gene, a PCR was performed by using a primer set of poxB_up_for (SEQ ID NO: 31) and poxB_dn_rev (SEQ ID NO:32). Additionally, in order to confirm the presence of any deletions in pta-ackA gene, a primer set of pta_up_for (SEQ ID NO: 33) and ackA_dn_rev (SEQ ID NO: 34) was used. In order to confirm the presence of any deletions in actA gene, a PCR was performed by using a primer set of actA_up_for (SEQ ID NO: 35) and actA_dn_rev (SEQ ID NO: 36).

### Example 2. Construction of CGL (Δldh, ΔpoxB, Δpta-ackA, ΔactA, pyc^{P458S})

A variant, (hereinafter, 'PYC^{Pa58S}'), in which proline, the 458^{th} amino acid of the pyruvate carboxylase of C. glutamicum ATCC 13032 (SEQ ID NO: 14), is replaced with serine, was prepared.

The variant was prepared by replacing a codon CCG, which encodes proline, the 458^{th} amino acid of PYC amino acid, with a codon TCG via overlap extension PCR.

Two PCR products were obtained from a genomic DNA of CGL ATCC 13032, one PCR product by using a primer set of pyc-F1 (SEQ ID NO: 37) and pyc-R1 (SEQ ID NO: 38), and the other PCR product by using a primer set of pyc-F2 (SEQ ID NO: 39) and pyc-R2 (SEQ ID NO: 40). A third PCR product was obtained by using the obtained two PCR products as a template and a primer set of pyc-F1 and pyc-R2. The third PCR product was cloned into a Xbal restriction site of a pK19mobsacB vector to construct a pK19mobsacB-pyc* vector.

The pK19mobsacB-pyc* vector was introduced into CGL (Δldh, ΔpoxB, Δpta-ackA, ΔactA) of Example 1. A PCR was performed by using a primer set of pyc-F1 and pyc-R2. The sequence of the resulting PCR product was analyzed, and the presence of replacement of pyc gene was examined.

### Example 3: Preparation of a bacterial strain expressing α-ketoglutarate decarboxylase (KGDC) and succinyl semialdehyde dehydrogenase (SSADH)

### (1) Construction of pGS_Ptuf::sucA:NCgl0463

pGS_Ptuf::sucA:NCgl0463 vector was constructed as follows in order to express KGDC gene (sucA, SEQ ID NO: 4) of E. coli, and SSADH gene (NCgl0463, SEQ ID NO: 65) of Corynebacterium glutamicum under the tuf promoter.

### 1) Construction of pGS EX4

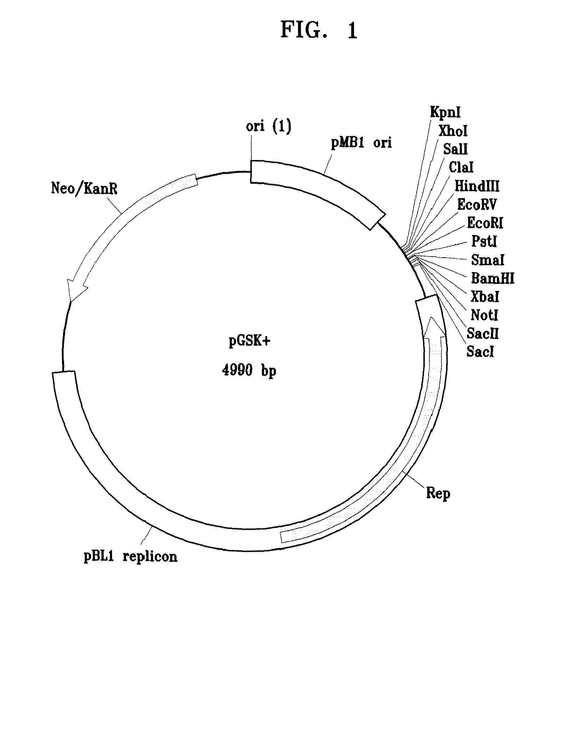

The following four PCR products were obtained by using Phusion High-Fidelity DNA Polymerase (New England Biolabs, cat.# M0530). A PCR was performed by using pET2 (GenBank accession number: AJ885178.1), i.e., a vector for screening a promoter of *Corynebacterium glutamicum,* as a template and by using a primer set of MD-616 (SEQ ID NO: 41) and MD-618 (SEQ ID NO: 42), and a primer set of MD-615 (SEQ ID NO: 43) and MD-617 (SEQ ID NO: 44). In addition, another PCR was performed by using pEGFP-C1 (Clontech) as a template, and by using a primer set of MD-619 (SEQ ID NO: 45) and MD-620 (SEQ ID NO: 46), and a primer set of LacZa-NR (SEQ ID NO: 47) and MD-404 (SEQ ID NO: 48). Each of the PCR products, i.e., 3010 bp, 854 bp, 809 bp, and 385 bp fragments, was cloned into a circular plasmid by using an In-Fusion EcoDry PCR Cloning Kit (Clontech, cat.# 639690). The cloned vector was transformed into a One Shot TOP10 Chemically Competent Cell (Invitrogen, cat.# C4040-06), cultured in LB medium containing 25 mg/L of kanamycin, and growing colonies were selected. The vector was recovered from the selected colonies, and the nucleotide sequence of the vector was confirmed by analyzing the entire length of the vector sequence. The vector was assigned as pGSK+ (FIG. 1).

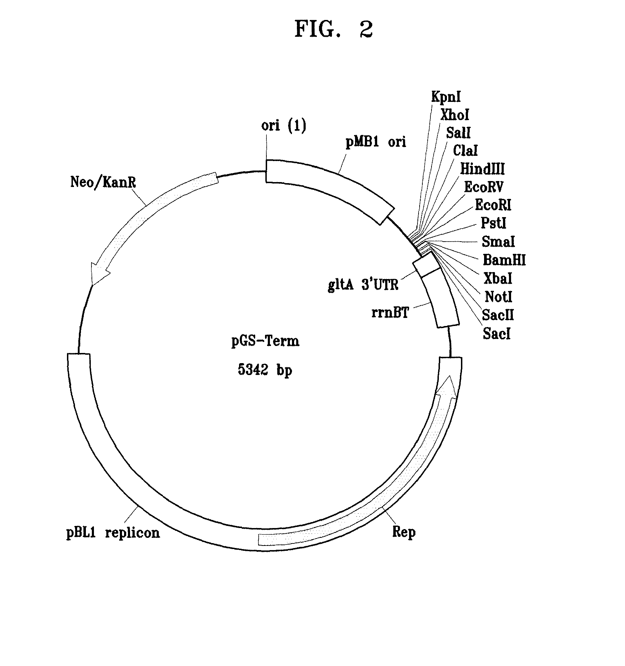

Additionally, 3'UTR of C. glutamicum gltA (NCgl0795) and rho-independent terminator of E. coli rrnB were inserted into the pGSK+ vector as follows. A 108 bp PCR product of gltA 3'UTR was obtained by performing a PCR by using the genomic DNA of C. glutamicum (ATCC13032) as a template, and by using a primer set of MD-627 (SEQ ID NO: 49) and MD-628 (SEQ ID NO: 50). Furthermore, a 292 bp PCR product of rrnB terminator was obtained by using the genomic DNA of E. coli (MG1655) and by using a primer set of MD-629 (SEQ ID NO: 51) and MD-630 (SEQ ID NO: 52). The two amplified fragments were inserted into pGSK+ digested with SacI by using an In-Fusion EcoDry PCR Cloning Kit (Clontech, cat.# 639690). The cloned vector was transformed into a One Shot TOP10 Chemically Competent Cell (Invitrogen, cat.# C4040-06), cultured in LB medium containing 25 mg/L of kanamycin, and growing colonies were selected. The vector was recovered from the selected colonies, and the nucleotide sequence of the vector was confirmed by analyzing the entire length of the vector sequence. The vector was assigned as pGS-Term (FIG. 2).

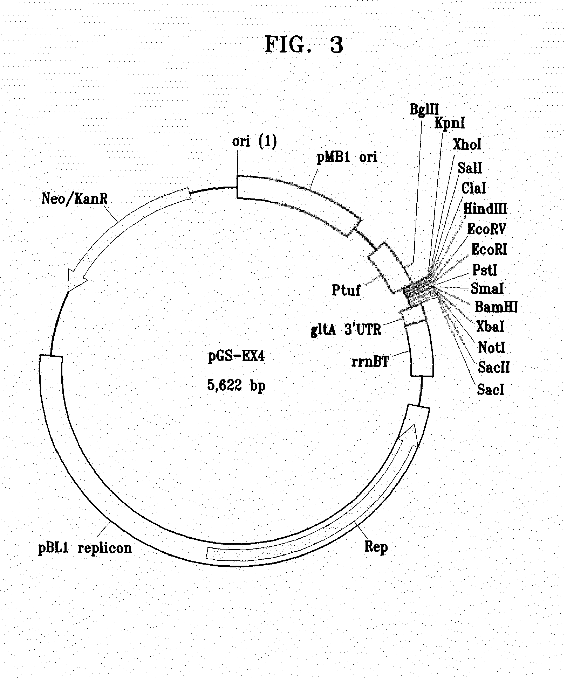

In addition, a Ptuf was obtained by using the genomic DNA of C. glutamicum ATCC 13032 as a template, and by using a primer set of Tuf-F (SEQ ID NO: 53) and Tuf-R (SEQ ID NO: 54). Ptuf is a promoter of tuf gene derived from C. glutamicum. The obtained Ptuf fragment was cloned into the Kpnl restriction site of the pGS-Term vector by using an In-Fusion® HD Cloning Kit (Clontech 639648) and pGS_EX4 vector was obtained therefrom (FIG. 3).

### 2) Construction of pGS Ptuf:sucA:NCgl0463

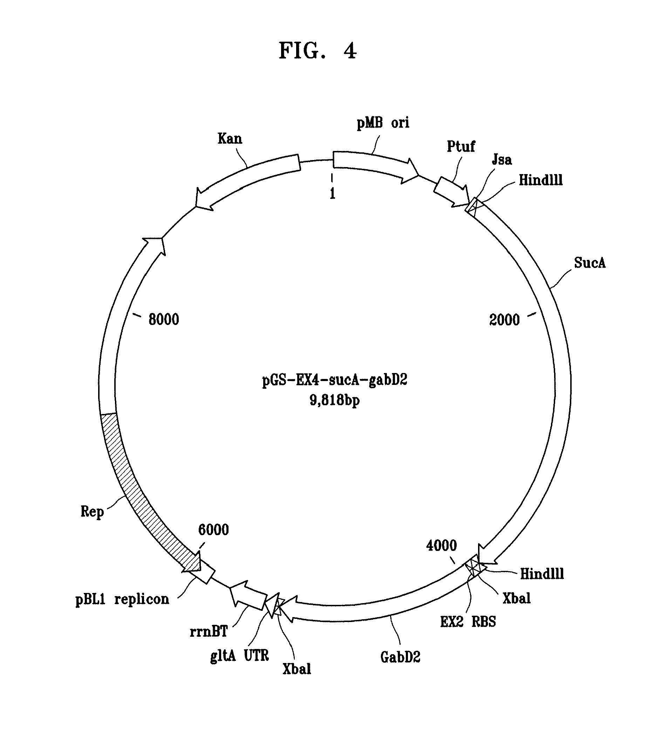

sucA gene was amplified by using the genomic DNA of MG1655, which belong to *E. coli* K12, as a template and by using a primer set of sucA-F (SEQ ID NO: 55) and sucA-R (SEQ ID NO: 56). Furthermore, the gabD1 (NCgl0463) gene was amplified by using the genomic DNA of C. glutamicum ATCC 13032 as a template, and by using a primer set of NCgl0463_RBS-F (SEQ ID NO: 57) and NCgl0463-R (SEQ ID NO: 58). Each of the amplified products was cloned into a Hindlll-Xbal restriction site of a pGS_EX4 vector, and pGS_Ptuf::sucA:NCgl0463 vector was obtained therefrom (FIG. 4). FIG. 4 shows a map of pGS_Ptuf::sucA:NCgl0463 vector.

### (2) Construction of CGL (Δldh, ΔpoxB, Δpta-ackA, ΔactA, pyc^{P458S}, pGS_Ptuf::sucA:NCgl0463)

pGS_Ptuf::sucA:NCgl0463 vector was introduced into a C. glutamicum ATCC 13032 (Δldh, ΔpoxB, Δpta-ackA, ΔactA, pyc^{P458S}) competent cell via electroporation. The resultant was plated out on LBHIS medium containing 25 µg/mL of kanamycin and incubated at 30°C. CGL (Δldh, ΔpoxB, Δpta-ackA, ΔactA, pyc^{P458S}, pGS_Ptuf::sucA:NCgl0463) was obtained by selecting the strains resistant to kanamycin.

The expressions of sucA and NCgl0463 were examined via RT-PCR by using a primer set of SucA-RTF (SEQ ID NO: 59) and SucA-RTR (SEQ ID NO: 60), and by using a primer set of NCgl0463-RTF (SEQ ID NO: 61) and NCgl0463-RTR (SEQ ID NO: 62). Forty cycles of RT-PCR were performed where each cycle consisted of denaturation at 95°C for 15 seconds, annealing at 60°C for 15 seconds, and extension at 72°C for 30 seconds. As a result, the expressions of sucA and NCgl0463 were confirmed.

### Example 4. Cultivation of bacterial strains prepared in Example 3 and production of succinic acid

The productivity of CGL (Δldh, ΔpoxB, Δpta-ackA, ΔactA, pyc^{P458S}, pGS_Ptuf::sucA:NCgl0463) on succinic acid was evaluated.

For seed culture, each bacterial strain was streaked on an active plate containing 5g/L of yeast extract, 10g/L of beef extract, 10 g/L of polypeptone, 5 g/L of NaCl and 20 g/L of agar, and incubated at 30°C for 48 hours. A single colony was inoculated to 5 mL of BHIS medium (37 g/L of brain-heart infusion broth, 91 g/L of D-sorbitol, pH 7.0), and cultured at 30°C until the OD₆₀₀ of the culture broth became 5.0.

1 mL of the thus obtain culture liquid was inoculated into a 250 mL flask containing 25 mL of BHIS medium. After centrifugation of the resulting culture liquid, the supernatant was discarded and microorganisms were collected, which were then washed with CGXII minimal medium. The minimal medium contains 20 g/L of (NH₄)₂SO₄, 5 g/L of urea, 1 g/L of KH₂PO₄, 1 g/L of K₂HPO₄, 0.25 g/L of MgSO₄·7H₂O, 10 mg/L of CaCl₂, 10 mg/L of FeSO₄·7H₂O, 0.1 mg/L of MnSO₄·H₂O, 1 mg/L of ZnSO₄·7H₂O, 0.2 mg/L of CuSO₄·5H₂O, 20 mg/L of NiCl₂·6H₂O, 0.2 mg/L of biotin, 42 g/L of 3- morpholinopropanesulfonic acid (MOPS) and 4%(w/v) of glucose.

Then, the washed cells were added into a 1-well plate containing 20 mL of CGXII minimal medium, which was not sealed but covered on its top, until the O.D. of the cells became 20. The plate was placed in an incubator (STX 40; Liconic instruments), where oxygen concentration is under control, kept at 30°C for 16 hours while maintaining the oxygen level in contact with the medium at 5%. The concentration of oxygen dissolved in the medium was 1.0 ppm. The sample was collected, centrifuged, and the concentrations of succinic acid and glucose of the resulting supernatant were analyzed via HPLC. Table 1 shows the analytical results.

**Table 1**

| Strain | Glucose consumption (g/L) | Succinate production (g/L) |
|---|---|---|
| S006/pGSEx4 | 39.9±0.6 | 0.36±0.08 |
| S006/Ptuf::sucA:gabD1 | 60.7±0.3 | 1.06±0.24 |

In Table 1, S006/pGSEx4 represents a control group strain formed by introducing pGSEx4 to the ATCC 13032 (Δldh, ΔpoxB, Δpta-ackA, ΔactA, pyc^{P458S}) strain, and S006/Ptuf::sucA:gabD1 represents an experimental group strain formed by the Ptuf:aucA:gabD1 vector to the same ATCC 13032 strain.

As shown in Table 1, the bacterial strain introduced with pGS_Ptuf::sucA:NCgl0463 showed about a three-fold increase in the production of succinic acid as compared to the control, a bacterial strain introduced with pGS_EX4. Accordingly, it was confirmed that the productivity of succinic acid of a bacterial cell in a microaerobic condition can be considerably increased by over-expression of KGDC and SSADH.

It should be understood that the exemplary embodiments described therein should be considered in a descriptive sense only and not for purposes of limitation. Descriptions of features or aspects within each embodiment should typically be considered as available for other similar features or aspects in other embodiments.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. Likewise, the expression "combination thereof" encompasses and shall individually disclose all possible combination. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Moreover, any combination of the above-described elements in all possible variations thereof is encompassed by the invention unless otherwise indicated herein or otherwise clearly contradicted by context.

## Claims

1. A genetically engineered bacterial cell, wherein activity of a pathway in the cell of converting α-ketoglutarate (AKG) into succinate semialdehyde (SSA) or activity of succinyl semialdehyde dehydrogenase (SSADH) in the cell is increased as compared to the activity in a parent cell, wherein the pathway of converting AKG into SSA includes α-ketoglutarate decarboxylase (KGDC); glutamate:succinate semialdehyde transaminase and glutamate decarboxylase; or a combination thereof, and the KGDC catalyzes the conversion of α-ketoglutarate into SSA, and the SSADH catalyzes the conversion of SSA into succinic acid.

2. The genetically engineered bacterial cell according to claim 1, wherein the cell produces succinic acid, preferably under microaerobic conditions.

3. The genetically engineered bacterial cell according to claim 1 or 2, wherein the cell is a *Corynebacterium* cell.

4. The genetically engineered bacterial cell according to any one of claims 1 to 3, wherein the activity is increased by a mutation in the amino acid sequence of KGDC, glutamate:succinate semialdehyde transaminase, glutamate decarboxylase, SSADH, or a combination thereof.

5. The genetically engineered bacterial cell according to any one of claims 1 to 4, wherein the activity is increased (a) by an increase in copy number of a gene encoding KGDC, a gene encoding glutamate:succinate semialdehyde transaminase, a gene encoding glutamate decarboxylase, a gene encoding SSADH, or a combination thereof, as compared to a parent cell, and/or (b) by increasing the expression of a gene encoding KGDC, a gene encoding glutamate:succinate semialdehyde transaminase, a gene encoding glutamate decarboxylase, a gene encoding SSADH, or a combination thereof, as compared to a parent cell.

6. The genetically engineered bacterial cell according to claim 5, wherein the bacterial cell comprises an exogenous gene encoding KGDC, an exogenous gene encoding glutamate:succinate semialdehyde transaminase, an exogenous gene encoding glutamate decarboxylase, an exogenous gene encoding SSADH, or a combination thereof; and/or wherein the copy number is increased by amplification of endogenous gene(s).

7. The genetically engineered bacterial cell according to claim 5 or 6, wherein the expression of the gene(s) is increased by modification of regulatory sequences for expressing the gene(s).

8. The genetically engineered bacterial cell according to any one of claims 1 to 7, wherein the KGDC is from *Corynebacterium., Rhodococcus* sp., *Mycobacterium* sp., or *Escherichia* sp., and/or the succinyl semialdehyde dehydrogenase is from *Corynebacterium., Rhodococcus* sp., *Gordonia* sp., *Mycobacterium* sp., or *Escherichia* sp..

9. The genetically engineered bacterial cell according to any one of claims 1 to 8, wherein (a) the KGDC has an amino acid sequence with a sequence identity of 95% or higher with an amino acid sequence of SEQ ID NO: 1 or SEQ ID NO: 2, and/or (b) the succinyl semialdehyde dehydrogenase has an amino acid sequence with a sequence identity of 95% or higher with an amino acid sequence of SEQ ID NO: 5, an amino acid sequence with a sequence identity of 95% or higher with an amino acid sequence of SEQ ID NO: 63, or an amino acid sequence with a sequence identity of 95% or higher with an amino acid sequence of SEQ ID NO: 64.

10. The genetically engineered bacterial cell according to any one of claims 1 to 9 comprising an inactivated or attenuated gene encoding succinyl-CoA synthetase, an inactivated or attenuated L-lactate dehydrogenase gene, an inactivated or attenuated pyruvate oxidase gene, an inactivated or attenuated phosphotransacetylase gene, an inactivated or attenuated acetate kinase gene, an inactivated or attenuated acetate CoA transferase gene, or a combination thereof.

11. The genetically engineered cell of claim 10, wherein the gene encoding succinyl-CoA synthetase comprises an amino acid sequence with 95% or higher identity with an amino acid sequence of SEQ ID NO: 7 or SEQ ID NO: 8.

12. The genetically engineered bacterial cell according to any one of claims 1 to 11, wherein pyruvate carboxylase activity catalyzing the conversion of pyruvate into oxaloacetate is increased in the genetically engineered bacterial cell as compared to a parent cell, wherein the pyruvate carboxylase preferably comprises SEQ ID NO: 14 with amino acid substitution P458S.

13. A method for producing succinic acid comprising:
culturing, preferably under microaerobic conditions, the cell according to any one of claims 1 to 12, whereby the cell produces succinic acid; and
recovering succinic acid from the culture.

14. A method of improving succinic acid production by a bacterial cell, the method comprising increasing the activity of succinyl semialdehyde dehydrogenase (SSADH), α-ketoglutarate decarboxylase (KGDC), glutamate:succinate semialdehyde transaminase, glutamate decarboxylase, or a combination thereof, in the cell.

15. The method of claim 14, wherein the method comprises increasing the copy number of a gene encoding succinyl semialdehyde dehydrogenase (SSADH), α-ketoglutarate decarboxylase (KGDC), glutamate:succinate semialdehyde transaminase glutamate decarboxylase, or a combination thereof, in the cell.
